Europäisches Patentamt

European Patent Office

Office européen des brevets

(11) Publication number: **0 423 890 A2**

# EUROPEAN PATENT APPLICATION

(21) Application number: 90202731.7

(22) Date of filing: 25.07.85

(51) Int. Cl.⁵: **C12N 15/00, C12N 1/16,**
C11D 3/386, C11D 3/395,
C12P 21/02, C07H 21/04,
C12N 9/02

The applicant has filed a statement in accordance with Rule 28 (4) EPC (issue of a sample only to an expert). Accession number(s) of the deposit(s): CBS 7171, CBS 7172 and ATCC 34438.

(30) Priority: 27.07.84 EP 84201114
07.02.85 GB 8503160

(43) Date of publication of application:
24.04.91 Bulletin 91/17

(60) Publication number of the earlier application in accordance with Art.76 EPC: **0 173 378**

(84) Designated Contracting States:
**AT BE CH DE FR GB IT LI NL SE**

(71) Applicant: **UNILEVER NV**
**Burgemeester s'Jacobplein 1 P.O. Box 760**
**NL-3000 DK Rotterdam(NL)**
(84) **BE CH DE FR IT LI NL SE AT**

Applicant: **UNILEVER PLC**
**Unilever House Blackfriars P.O. Box 68**
**London EC4P 4BQ(GB)**
(84) **GB**

(72) Inventor: **Ledeboer, Adrianus Marinus**
**Unilever Reseach Lab., Olivier van Noortlaan**
**120**
**Vlaardingen(NL)**
Inventor: **Maat, Jan**
**Unilever Reseach Lab., Olivier van Noortlaan**
**120**
**Vlaardingen(NL)**
Inventor: **Verrips, Cornelis Theodorus**
**Unilever Reseach Lab., Olivier van Noortlaan**
**120**
**Vlaardingen(NL)**
Inventor: **Visser, Christiaan**
**Unilever Reseach Lab., Olivier van Noortlaan**
**120**
**Vlaardingen(NL)**
Inventor: **Janowicz, Zbigniew Alojzy**
**Adalbert-Stifter-Strasse 49**
**W-4006 Erkrath-Unterfeldhaus(DE)**
Inventor: **Hollenberg, Cornelis Petrus**
**Chopin-Strasse 7**
**W-4000 Düsseldorf(DE)**

(74) Representative: **van der Toorren, Johannes,**
**Drs. et al**
**UNILEVER N.V. Patent Division Postbus 137**
**NL-3130 AC Vlaardingen(NL)**

(54) Use of oxioreductases in bleaching and/or detergent compositions and their preparation by microorganisms engineered by recombinant DNA technology.

(57) The structural genes and their regulatory DNA sequences of an alcohol oxidase (MOX) and a dihydroxyacetone synthase (DHAS) of *Hansenula polymorpha* have been isolated and the nucleotide sequences determined. The invention relates to the use of the MOX gene, as well as the use of the regulatory DNA sequences of MOX and/or DAS in combination with the MOX gene, optionally after modification thereof, or other oxidase genes, or other genes, to produce engineered microorganisms, in particular yeasts.

Said engineered microorganisms can produce oxidases or other enzymes in yields that allow industrial application on a large scale.

Moreover, said engineered microorganisms can produce oxidases having improved properties with respect to their application in oxidation reactions and/or in bleaching and detergent products.

# USE OF OXIDOREDUCTASES IN BLEACHING AND/OR DETERGENT COMPOSITIONS AND THEIR PREPARATION BY MICROORGANISMS ENGINEERED BY RECOMBINANT DNA TECHNOLOGY

The present invention relates to a process for microbiologically preparing oxidoreductases, use of these enzymes in bleaching and/or detergent compositions, as well as to microorganisms transformed by DNA sequences coding for an oxidoreductase and optionally for a dihydroxyacetone synthase-enzyme, and H . polymorpha alcohol oxidase and/or dihydroxyacetone synthase regulation sequences, the microorganisms being suitable for use in the process.

Oxidoreductases, especially those which use oxygen as electron acceptor, are enzymes suitable for use in bleaching and/or detergent compositions in which they can be used for the in situ formation of bleaching agents, e.g. $H_2O_2$, during the washing or bleaching process. See for example

- GB-PS 1 225 713 (Colgate-Palmolive Company), in which the use of a mixture of glucose and glucose oxidase and other ingredients in a dry powdered detergent composition has been described,
- DE-PA 2 557 623 (Henkel & Cie GmbH), in which the use of a $C_1$ to $C_3$ alkanol and alcohol oxidase, or galactose and galactose-oxidase, or uric acid and uratoxidase, and other ingredients in a dry detergent composition having bleaching properties has been described, and
- GB-PA 2 101 167 (Unilever PLC) in which the use of a $C_1$ to $C_4$ alkanol and a $C_1$ to $C_4$ alkanol oxidase in a liquid bleach and/or detergent composition has been described,

wherein the alkanol and the enzyme are incapable of substantial interaction until the composition is diluted with water, and/or has come into contact with sufficient oxygen.

Up to now natural oxidase-enzymes cannot be produced at a cost price that allows industrial application on a large scale, e.g. detergent products. Moreover, the oxidase-enzymes have to act under non-physiological conditions when used in detergent and bleaching products. Further the natural oxidases that have been investigated for use in detergent compositions are accompanied by the natural catalase-enzyme which decomposes almost immediately the peroxide(s) formed, so that no effective bleaching is obtained. Thus a need exists for oxidase-enzymes that are more suitable for use under the conditions of manufacture and use of detergent and bleaching products.

For an economically feasible production of these oxidases it is further required to reach a yield of these enzymes in fermentation processes in the order of that of alcohol oxidase of H . polymorpha , which is up to 20% of the cellular protein (van Dijken et al., 1976).

One way of finding new microorganisms producing enzymes in higher amounts or finding new oxidase-enzymes having improved properties is to check all sorts of microorganisms and try to isolate the relevant oxidases, which are then checked for their abilities to generate peroxides and their stabilities under the conditions of manufacture and use of detergent and bleaching products. One can hope that some day a suitable enzyme will be found, but the chance of success is unpredictable and probably very low.

Another way is to apply another trial and error method of crossing the natural microorganisms producing these oxidases by classical genetic techniques, in the hope that some day one will find a more productive microorganism or a more suitable enzyme, but again the chance of success is rather low.

Clearly, a need exists for a method for preparing oxidase-enzymes in higher yield and/or without the concomitant formation of catalase and/or having improved properties during storage and/or use in e.g. bleach and/or detergent compositions. The problem of trial and error can be overcome by a process for preparing an oxidase-enzyme by culturing a microorganism under suitable conditions, and preferably concentrating the enzyme and collecting the concentrated enzyme in a manner known per se , which process is characterized in that a microorganism is used that has been obtained by recombinant DNA technology and which is capable of producing said oxidase-enzyme.

The microorganisms suitable for use in a process for preparing an oxidase-enzyme can be obtained by recombinant DNA technology, whereby a microorganism is transformed by a DNA sequence coding for an oxidase-enzyme (so-called structural gene) together with one or more other DNA sequences which regulate the expression of the structural gene in a particular microorganism or group of organisms, either via introduction of an episomal vector containing said sequences or via a vector containing said sequences which is also equipped with DNA sequences capable of being integrated into the chromosome of the microorganism.

The determination of a structural gene coding for the enzyme alcohol oxidase (EC 1.1.3.13) originating from H . polymorpha together with its regulatory 5'- and 3'-flanking regions will be described as an example of the invention without the scope of the invention being limited to this example. The spirit of the invention is also applicable to the isolation of DNA sequences of other oxidase-enzymes such as glycerol oxidase, glucose oxidase, D-amino acid oxidase etc.; the incorporation of the DNA sequences or modifications thereof into the genome of microorganisms or into episomal vectors used for transforming microorganisms and the culturing of the transformed microorganisms so obtained as such or for producing the desired oxidase-enzymes, as well as the use of these enzymes in bleaching compositions containing them.

Although the microorganisms to be used can be bacteria, e.g. of the genus Bacillus , as well as moulds, the use of yeasts is preferred for technological and economical reasons. In particular a mould or yeast can be selected from the genera Aspergillus , Candida , Geotrichum , Hansenula , Lenzites , Nadsonia , Pichia , Poria , Polyporus , Saccharomyces , Sporobolomyces , Torulopsis , Trichosporon and Zendera , more particularly from the species A . japonicus , A . niger , A . oryzae , C . boidinii , H . polymorpha , pichia pastoris and Kloeckera sp. 2201. The latter name is sometimes used instead of C . Boidinii .

Many $C_1$-utilizing yeasts have been isolated during the last decade, and for Hansenula polymorpha and Candida boidinii the methanol metabolism has been studied extensively (for a review see Veenhuis et al., 1983).

The first step in this metabolism is the oxidation of methanol to formaldehyde and $H_2O_2$ catalyzed by MOX. Formaldehyde is oxidized further by the action of formaldehyde dehydrogenase and formate dehydrogenase. $H_2O_2$ is split into water and oxygen by catalase.

Alternatively, methanol is assimilated into cellular material. After its conversion into formaldehyde, this product is fixed through the xylulose monophosphate pathway into carbohydrates. Dihydroxyacetone synthase (DHAS) plays a crucial role in this assimilation process.

The appearance of MOX, formate dehydrogenase, formaldehyde dehydrogenase, DHAS and catalase is subject to glucose repression, e.g. on 0.5% glucose. However, synthesis of MOX is derepressed by growth in low concentrations of glucose (0.1%), contrary to the synthesis of DHAS, which is still fully repressed under these conditions (Roggenkamp et al., 1984).

Regulation, i.e. the possibility to switch "on" or "off" of the gene for the polypeptide concerned, is desirable, because it allows for biomass production, when desired, by selecting a suitable substrate, such as, for example melasse, and for production of the polypeptide concerned, when desired, by using methanol or mixtures of methanol, and other carbon sources. Methanol is a rather cheap substrate, so the polypeptide production may be carried out in a very economical way.

After derepression of the gene coding for alcohol oxidase (MOX) by growth on methanol, large microbodies, the peroxisomes are formed. While glucose-grown cells contain only a small peroxisome, up to 80% of the internal volume of the cell is replaced by peroxisomes in the derepressed state. The conversion of methanol into formaldehyde and $H_2O_2$ as well as the degradation of $H_2O_2$ has been shown to occur in these peroxisomes, while further oxidation or assimilation of formaldehyde most probably occurs in the cytoplasm. This process is a perfect example of compartmentalization of toxic pro ducts, of a strong co-ordinate derepression of several cellular processes and of the selective translocation of at least two of the enzymes involved in this process.

Most of the enzymes involved in the methanol metabolism have been purified and characterized (Sahm, 1977, Bystrykh et al, 1981). Especially methanol oxidase (EC 1.1.3.13) has been studied in detail. It is an octamer consisting of identical monomers with an $M_r$ value of about 74 kd and it contains FAD as a prosthetic group. Up to now no cleavable signal sequence for translocation could be detected, as concluded from electroelephoresis studies with in vivo and in vitro synthesized products (Roa and Blobel, 1983) or from in vitro synthesis in the presence of microsomal membranes (Roggenkamp et al., 1984).

Under derepressed conditions, up to 20% of the cellular protein consists of MOX.

## Materials and methods

### a) Microorganisms and cultivation conditions

Hansenula polymorpha CBS 4732 was obtained from Dr J.P. Van Dijken (University of Technology, Delft, The Netherlands). Cells were grown at 37° C in 1 litre Erlenmeyer flasks containing 300 ml minimal medium (Veenhuis et al., 1978), supplemented with 0.5% (v/v) methanol or 0.5% (v/v) ethanol as indicated. Phage lambda L47.1 and the P2 lysogenic E . coli K12 strain Q 364 were obtained from Dr P. van der Elsen (Free University of Amsterdam, The Netherlands) and propagated as described (Loenen and Brammar, 1980).

E . coli K12 strains BHB 2600, BHB 2688 and BHB 2690 (Hohn, 1979) were obtained from Dr. M. van Montagu (University of Gent, Belgium), while E . coli K12 strain JM 101.7118 and the M13 derivatives M13 mp 8, 9, 18 and 19 were obtained from Bethesda Research Laboratories Inc. (Gaithersburg, MD, U.S.A.).

b) Enzymes

All enzymes used were obtained from Amersham International PLC, Amersham, U.K., except alpha-helicase which was obtained from Pharm Industrie, Clichy, France. Enzyme incubations were performed according to the instructions of the manufacturer.
ATP:RNA adenyl transferase was purified as described by Edens et al. (1982).


c) Other materials

[$^{35}$S] methionine, [alpha-$^{35}$S] dATP, [alpha-$^{32}$P] dNTP's, [alpha-$^{32}$P] ATP and [gamma-$^{32}$P] ATP were obtained from Amersham International PLC, Amersham, U.K.
Nitrobenzyloxy-methyl (NBM) paper was obtained from Schleicher and Schuell, and converted into the diazo form (DBM) according to the instructions of the manufacturer.
Nitrocellulose filters (type HATF) were obtained from Millipore.


RNA isolation, fractionation and analysis

Hansenula polymorpha cells were grown to midexponential phase, either in the presence of methanol or ethanol. The cells were disrupted by forcing them repeatedly through a French Press at 16 000 psi, in a buffer containing 10 mM Tris-HCl pH 8, 5 mM MgCl$_2$, 1% NaCl, 6% para-aminosalicylic acid, 1% sodium do decylsulphate (SDS) and 5% phenol. The purification of polyadenylated RNA was subsequently performed, as described previously (Edens et al., 1982). One gram cells yielded four mg total RNA and 0.1 mg polyadenylated RNA. Five microgram samples of total RNA or polyadenylated RNA were radioactively labelled at their 3'-ends with ATP:RNA adenyl transferase and [alpha-$^{32}$-P] ATP, and subsequently separated on a 2.5% polyacrylamide gel containing 7 M urea (Edens et al., 1982). For the preparative isolation of a specific mRNA fraction, 40 micrograms polyadenylated RNA was mixed with four micrograms of labelled polyadenylated RNA and separated on the denaturing polyacrylamide gel. The radioactive 2.4 kb RNA class was eluted from slices of the gel and freed from impurities by centrifugation through a 5-30% glycerol gradient in 100 mM NaCl, 10 mM Tris-HCl pH 7.5, 1 mM EDTA and 0.1% SDS for 15 h at 24 000 rev./min. in a Beckmann centrifuge using an SW 60 rotor at 20° C. The radioactive fractions were pooled and precipitated with ethanol. Polyadenylated RNA was translated in vitro in a rabbit reticulocyte lysate according to Pelham and Jackson (1976), using [$^{35}$S] methionine as a precursor. The translation products were immuno-precipitated with MOX antiserum as described by Valerio et al. (1983).


cDNA synthesis

One third of the RNA fraction, isolated from the polyacrylamide gel, was used to procure a radioactive cDNA with reverse transcriptase (Edens et al., 1982). Using [alpha-$^{32}$P] dATP and [alpha-$^{32}$P] dCTP of a high specific activity (more than 3000 Ci/mM), 20 000 cpm of high molecular weight cDNA was formed during 1 h at 42° C in the presence of human placental ribonuclease inhibitor.


DNA isolation

Ten g of Hansenula polymorpha cells were washed with 1 M sorbitol and resuspended in 100 ml 1.2 M sorbitol, 10 mM EDTA and 100 mM citric acid pH 5.8, to which 100 microliter beta-mercapto-ethanol was added. Cells were spheroplasted by incubation with 500 mg alpha-helicase for 1 h at 30° C. Spheroplasts were collected by centrifugation at 4000 rev./min. in a Sorvall GSA rotor, resuspended in 40 ml 20 mM Tris-HCl pH 8, 50 mM EDTA and lysed by adding 2.5% SDS. Incompletely lysed cells were pelleted for 30 min. at 20 000 rev./min. in a Sorvall SS34 rotor and DNA was isolated from the viscous supernatant by centrifugation using a CsCl-ethidium bromide density gradient at 35 000 rev./min. for 48 h in a Beckmann centrifuge using a 60 Ti rotor. 2 mg of DNA was isolated with a mean length of 30 kb.


Preparation of a clone bank in phage lambda L47.1

4

150 microgram Hansenula polymorpha DNA was partially digested with Sau 3AI and sedimented through a 10-40% sucrose gradient in 1 M NaCl, 20 mM Tris-HCl pH 8 and 5 mM EDTA for 22 h at 23 000 rev./min. in an SW 25 rotor. The gradient was fractionated and samples of the fractions were separated on a 0.6% agarose gel in TBE buffer (89 mM Tris, 89 mM Boric acid, 2.5 mM EDTA).

Fractions that contained DNA of 5-20 kb were pooled and the DNA was precipitated with ethanol. Phage lambda L47.1 was grown, and its DNA was isolated as described by Ledeboer et al. (1984). The DNA was digested with Bam HI and arms were isolated by centrifugation through a potassium acetate gradient as described by Maniatis et al. (1982). Two microgram phage lambda DNA arms and 0.5 μg Sau 3AI digested Hansenula polymorpha DNA thus obtained were ligated and packaged in vitro using a protocol from Hohn (1979). Phages were plated on E . coli strain Q 364 to a plaque density of 20,000 pfu per 14 cm Petri dish. Plaques were blotted onto a nitrocellulose filter (Benton and Davis, 1977) and the blot was hybridized with the radioactive cDNA probe isolated as described above. Hybridization conditions were the same as described by Ledeboer et al. (1984) and hybridizing plaques were detected by autoradiography.

Isolation and partial amino acid sequence analysis of alcohol oxidase (MOX)

Hansenula polymorpha cells grown on methanol were disintegrated by ultrasonification and the cell debris was removed by centrifugation. The MOX-containing protein fraction was isolated by $(NH_4)_2SO_4$ precipitation . (40-60% saturation). After dialysis of the precipitate, MOX was separated from catalase and other proteins by ion-exchange chromatography (DEAE-Sepharose) and gel filtration (Sephacryl S-400). Antibodies against MOX were raised in rabbits by conventional methods using complete and incomplete Freund's adjuvants (Difco Lab, Detroit, U.S.A.). Sequence analysis of alcohol oxidase treated with performic acid was performed on a Beckman sequenator. Identification of the residues was done with HPLC. The amino acid composition was determined on a Chromaspek analyser (Rank Hilger, U.K.), using standard procedures and staining by ninhydrine. The carboxy terminal amino acid was determined as described by Ambler (1972).

Chemical synthesis of deoxyoligonucleotides

Deoxyoligonucleotides were synthesized on a Biosearch SAM I gene machine, using the phosphite technique (Matteucci and Caruthers, 1981). They were purified on 16% or 20% polyacrylamide gels in TBE.

Hybridization with deoxyoligonucleotide probes

The deoxyoligonucleotides were radioactively labelled with $T_4$-polynucleotide kinase and [gamma-$^{32}$P] ATP. The DNA of the MOX clones obtained was digested with different restriction enzymes, separated on 1% agarose gel and blotted onto DBM paper. Hybridizations were performed as described by Wallace et al. (1981).

DNA sequence analysis

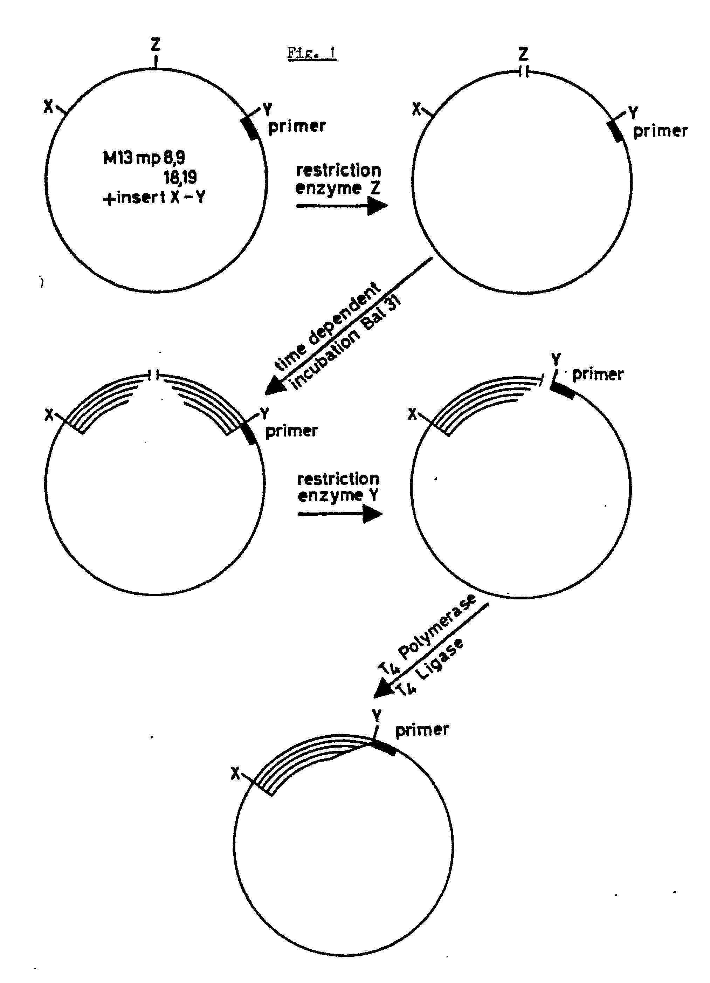

From clone 4 (see Example 1) containing the complete MOX gene, several subclones were made in phage M13mp-8, -9 or M13mp-18, -19 derivatives by standard techniques. Small subclones (less than 0.5 kb), cloned in two orientations, were sequenced directly from both sides. From the larger subclones, also cloned in two orientations, sequence data were obtained by an exonuclease Bal 31 digestion strategy (see Fig. 1). For each of both cloned orientations the RF M13 DNA is digested with a restriction enzyme that preferably cleaves only in the middle of the insert. Subsequently, both orientations of the clones were cut at this unique site, and digested with exonuclease Bal 31 at different time intervals. Incubation times and conditions were chosen such that about 100-150 nucleotides were eliminated during each time interval. Each fraction was digested subsequently with the restriction enzyme, recognizing the restriction site situated near the position at which the sequence reaction is primed in the M13 derivatives. Ends were made blunt end by incubation with $T_4$-polymerase and all dNTP's, and the whole mix was ligated under diluted conditions, thereby favouring the formation of internal RF molecules. The whole ligation mix was used to transform to E . coli strain JM 101-7118. From each time interval several plaques were picked up and

5

sequenced using recently described modifications of the Sanger sequencing protocol (Biggin et al., 1983).

The isolation of auxotrophic mutants

LEU-1 (CBS N° 7171) is an auxotrophic derivative of H . polymorpha strain NCYC 495 lacking β-isopropylmalate dehydrogenase activity. The isolation of this mutant has been described by Gleeson et al. (1984).

LR9 (CBS N° 7172) is an auxotrophic derivative of H . polymorpha ATCC 34438, lacking orotidine 5′-decarboxylase activity.

For the isolation, all procedures were carried out at 30°C instead of 37°C, which is the optimal temperature for growth of this yeast. Yeast cells were mutagenized with 3% ethylmethanesulphonate for 2 hr (Fink, 1970). The reaction was stopped with 6% sodium thiosulphate (final concentration) and the solution was incubated for another 10 min. Mutagenized cells were then washed once with $H_2O$ and incubated for 2 days on YEPD or YNB supplemented with uracil for segregation and enrichment of uracil-auxotrophs followed by a 15 hr cultivation on MM without nitrogen source. Finally a nystatin enrichment was employed for 12 hr on MM with a concentration of 10 μg antibiotic per ml. The treated cells were plated on YNB plates containing 200 μg uracil per ml and 0.8 mg 5-fluoroorotic acid (Boeke et al., 1984). Usually $10^6$ cells were plated on a single plate. Resistant colonies were picked after 3 days of incubation, replica plated twice on YNB plates to establish the auxotrophy. From the auxotrophic mutants ura⁻ cells were isolated. Alternatively, 1.5 x $10^6$ yeast cells were incubated in one ml of YNB liquid medium supplemented with 200 μg of uracil and 0.8 mg of 5-fluoroorotic acid. After incubation of 2 days, the treated cells were plated on YNB containing uracil, replica-plated twice on YNB and analysed as described above.

Such resistant mutants have been shown to be uracil auxotrophs affected at the URA3 or the URA5 locus in S . cerevisiae (F. Lacroute, personal communication). Of about 600 resistant colonies of H . polymorpha tested, 52 exhibited a uracil phenotype. Since URA3 and URA5 mutations in S . cerevisiae lack orotidine 5′-decarboxylase and orotidine 5′-phosphate pyrophosphorylase, respectively (Jones and Fink, 1982), the obtained uracil auxotrophs of H . polymorpha were tested for both enzymatic activities (Lieberman et al., 1955). Mutants affected in either of the two enzymes were found (Table I). They have been designated odcl and oppl mutants, respectively. The odcl mutants exhibit adequate low reversion frequencies (Table II) and thus are suitable for transformation purposes by complementation.

Isolation of autonomous replication sequences (HARS) from H. polymorpha

Chromosomal DNA from H . polymorpha was partially digested either with Sal I or Bam HI and ligated into the single Sal I and Bam HI site of the integrative plasmid YIp5, respectively. The ligation mixture was used to transform E . coli 490 to ampicillin resistance. YIp5 is an integrative plasmid containing the URA3 gene as a selective marker (Stinchcomb et al., 1980).

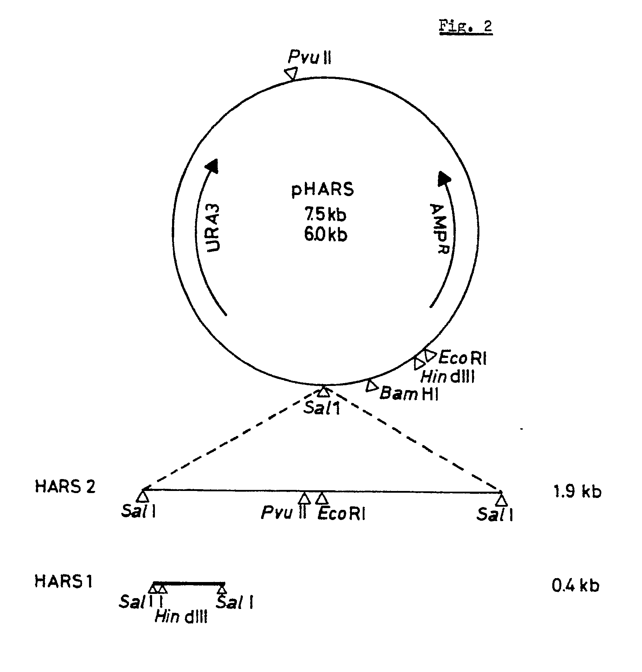

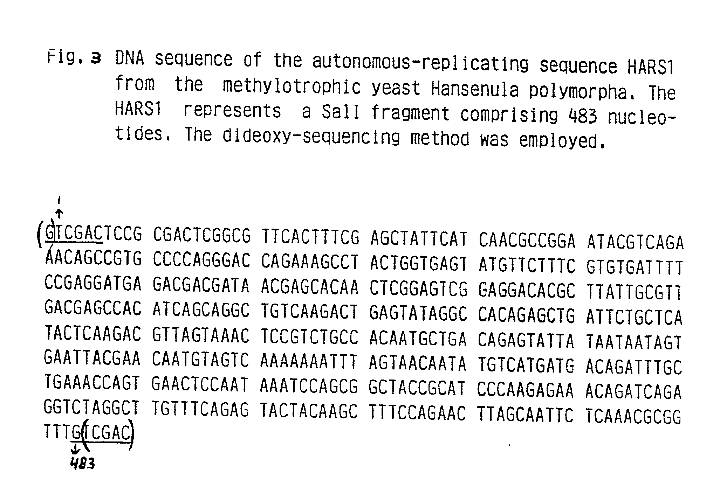

The plasmid pool of H . polymorpha Sal I clones was used to transform H . polymorpha mutant LR9. A total of 27 transformants was obtained being also positive in the β-lactamase assay. From all of them, plasmids could be recovered after transformation of E . coli 490 with yeast minilysates. Restriction analysis of the plasmids revealed that most of the inserts show the same pattern. The two different plasmids, pHARS1 and pHARS2, containing inserts of 0.4 and 1.6 kb respectively, were used for further studies (Fig. 2). Both plasmids transform H . polymorpha mutant LR9 with a frequency of about 500-1,500 transformants per μg of DNA using the transformation procedure of intact cells treated with polyethyleneglycol. Southern analysis of the H . polymorpha transformants after retransformation with pHARS1 and pHARS2 recovered from E . coli plasmid preparations shows the expected plasmid bands and thus excludes integration of the URA3 gene as a cause of the uracil protrophy. Therefore, we conclude that the HARS sequences like ARS1 (Stinchcomb et al., 1982) allow autonomous replication in H . polymorpha . Neither HARS1 nor HARS2 enabled autonomous replication in S . cerevisiae . HARS1 was sequenced completely as shown in Fig. 3.

Estimation of plasmid copy number in H. polymorpha transformants

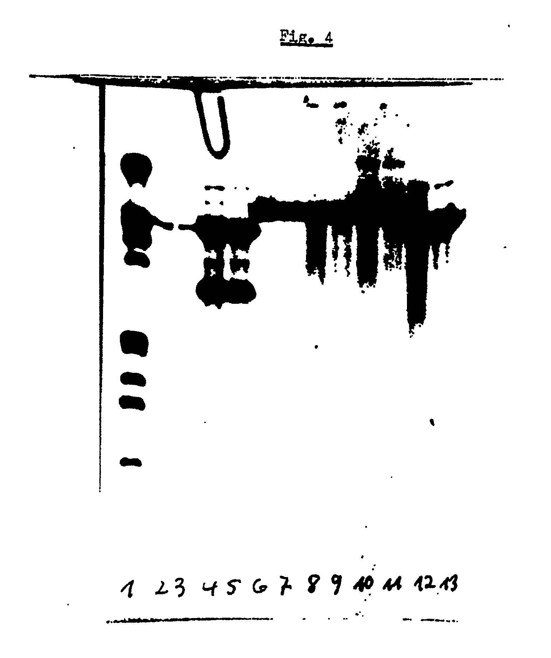

The copy number of plasmids conferring autonomous replication in H . polymorpha either by ARS sequences or by HARS sequences was estimated by Southern blot analysis (Fig. 4). For comparison, plasmid YRP17 in S . cerevisiae (Fig. 4, lanes 6, 7), which has a copy number of 5-10 per cell (Struhl et al.,

6

1979) and the high copy number plasmid pRB58 in S . cerevisiae (Fig. 4, lanes 4, 5) with about 30-50 copies per cell were used. YRP17 is a URA3-containing yeast plasmid, bearing an ARS sequence (Stinchcomb et al., 1982), while pRB58 is a 2 $\mu$m derivative containing the URA3 gene (Carlson and Botstein, 1982). A Kluyveromyces lactis transformant carrying 2 integrated copies of pBR pBR322 was used as a control (Fig. 4, lanes 2, 3). The intensity of staining in the autoradiogram reveals that the plasmid YRP17 in H . polymorpha has practically the same copy number as in S . cerevisiae , whereas plasmids pHARS-1 and pHARS-2 show a copy number which is in the range of about 30-40 copies per cell like pBR58 in S . cerevisiae . This proves once more the autonomously replicating character of the HARS sequence.


Transformation procedures

Several protocols were used.

a) H . polymorpha strain LEU-1 was transformed using a procedure adapted from Beggs (1978). The strain was grown at 37° C with vigorous aeration in 500 ml YEPD liquid medium up to an $OD_{600}$ of 0.5. The cells were harvested, washed with 20 ml distilled water and resuspended in 20 ml 1.2 M sorbitol, 25 mM EDTA pH 8.0, 150 mM DTT and incubated at room temperature for 15 minutes. Cells were collected by centrifugation and taken up in 20 ml 1.2 M sorbitol, 0.01 M EDTA, 0.1 M sodium citrate pH 5.8 and 2% v/v beta-glucuronidase solution (Sigma 1500000 units/ml) and incubated at 37° C for 105 minutes. After 1 hr, the final concentration of beta-glucuronidase was brought to 4% v/v. For transformation, 3 ml aliquots of the protoplasts were added to 7 ml of ice cold 1.2 M sorbitol, 10 mM Tris-HCl pH 7. Protoplasts were harvested by centrifugation at 2000 rpm for 5 minutes and washed three times in ice cold sorbitol buffer. Washed cells were resuspended in 0.2 ml 1.2 M sorbitol, 10 mM CaCl2, 10 mM Tris-HCl pH 7 on ice. 2 $\mu$g of YEP13 DNA - an autonomous replicating S . cerevisiae plasmid consisting of the LEU2 gene of S . cerevisiae and the 2 micron-ori (Broach et al., 1979) - were added to 100 ml of cells and incubated at room temperature. 0.5 ml of a solution of 20% PEG 4000 in 10 mM CaCl2, 10 mM Tris-HCl pH 7.5 was added and the whole mixture was incubated for 2 minutes at room temperature. cells were collected by brief (5 sec.) centrifugation in an MSG microfuge set at high speed and resuspended in 0.1 ml YEPD 1.2 M sorbitol pH 7.0, and incubated for 15 minutes at room temperature. The cells were plated directly by surface spreading on plates containing 2% Difco agar, 2% glucose, 0.67% Difco yeast nitrogen base and 20 mg/l of each of L-adenine Hemisulphate, methionine, uracil, histidine, tryptophan, lysine and 1.2 M sorbitol. Leu$^+$ transformants appear after 5 days incubation at 37° C with a frequency of 50 colonies/ $\mu$g DNA, while no transformants appear if no DNA is added.

b) Alternatively, H . polymorpha LEU-1 was transformed with YEP13, using a procedure adapted from Das et al. (1984). Exponentially growing cells were grown up to an $OD_{600}$ of 0.4, washed in TE buffer (50 mM Tris-HCl pH 8.0, 1 mM EDTA) and resuspended in 20 ml TE buffer. 0.5 ml cells were incubated with 0.5 ml 0.2 M LiCl for 1 hr at 30° C. To 100 ml of these cells 4 $\mu$g YEP13 in 20 ml TE buffer was added and the sample was incubated for a further 30 minutes at 30° C. An equal volume of 70% v/v PEG 4000 was added and the mixture was incubated for 1 hr at 30° C, followed by 5 min. at 42° C. After addition of 1 ml H2O, cells were collected by a brief centrifugation as described under a), washed twice with H2O and resuspended in 0.1 ml YEPD 1.2 M sorbitol and incubated for 15 minutes at room temperature. Cells were plated as described. Leu$^+$ transformants appear with a frequency of 30/ $\mu$g DNA.

c) The H . polymorpha URA mutant LR9 was transformed with YRP17, a plasmid containing the URA3 gene of S . cerevisiae as a selective marker and an autonomously replicating sequence (ARS) for S . cerevisiae (Stinchcomb et al, 1982). Using the protoplast method described by Beggs (1978), 2-5 transformants/ $\mu$g DNA were obtained. This number was enlarged, using the LiSO4 method of Ito et al. (1983), up to 15-20 transformants per $\mu$g of DNA. However, the best procedure was the procedure described by Klebe et al. (1983), using intact cells treated with PEG 4000. Up to 300 transformants were obtained per $\mu$g DNA. The LiSO4 procedure, as well as the Klebe procedure, was performed at 37° C.

Transformation of H . polymorpha based on autonomous replication of the vector was indicated by two characteristics: (1) the instability of the uracil$^+$ phenotype. After growth of transformants on YEPD for ten generations, more than 99% had lost the ability to grow on selective medium (Table II). (2) Autonomous replication was further ascertained by transforming E . coli cells with yeast minilysates and retransformation of H . polymorpha . Subsequent Southern analysis showed the presence of the expected plasmid.

H . polymorpha LR9 could not be transformed with pRB58, or with pHH85, constructed by insertion of the whole 2 micron circle DNA (Hollenberg, 1982) into the Pst I site of the ampicillin gene of plasmid YIP5. YIP5, containing the DNA sequence of HARS1 or HARS2, was transferred to H . polymorpha LR9 using the Klebe protocol with a frequency of 500-1500 transformants per μg of DNA. Thus, transformation frequency is 2-5 times higher than described above, using the heterologous ARS 1 in YRP17 of S . cerevisiae . Similarly, the stability of the HARS plasmid in transformants is slightly higher than the ARS 1 plasmid (Table II).

Transformation of H. polymorpha by integration of the URA3 gene from S. cerevisiae

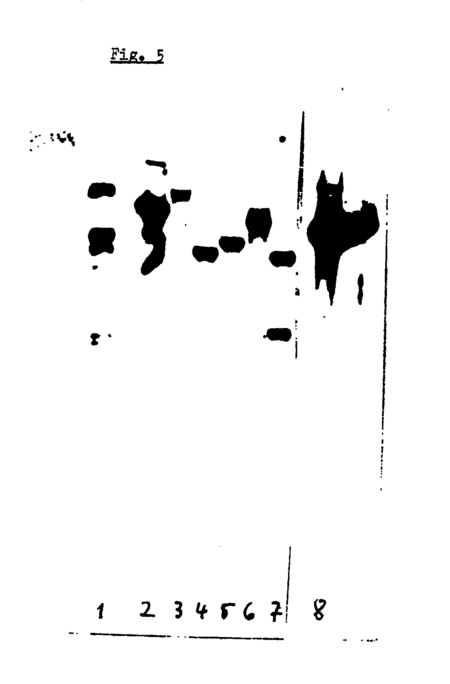

The URA3 gene of S . cerevisiae shows no homology to the ODC gene in H . polymorpha , as revealed by Southern hybridisation of nick-translated YIp5 plasmid DNA to chromosomal DNA of H . polymorpha . Therefore, low-frequency integration of the URA3 gene at random sites of the H . polymorpha genome had to be anticipated. Transformation of mutant LR9 with the integrative vector YIp5 resulted in 30-40 colonies per μg of DNA on YNB plates using the polyethyleneglycol method, whereas no transformants were obtained in the control experiment using YIp5 for transformation of S . cerevisiae mutant YNN27. Analysis of 38 transformants revealed 4 stable integrants after growth on non-selective medium. The integration event was further demonstrated by Southern analysis (Fig. 5).

A second procedure for generating integration of the URA3 gene into chromosomal DNA of H . polymorpha was performed by enrichment of stable Ura$^+$ transformants from transformants carrying plasmid pHARS1. Transformants were grown in liquid YEPD up to a density of $10^9$ cells per ml. An aliquot containing $5 \times 10^6$ cells was used to inoculate 100 ml of fresh medium and was grown up to a cell density of $10^9$ per ml. The procedure was repeated until about 100 generations had been reached. Since the reversion rate of mutant LR9 is $2 \times 10^{-9}$ and the frequency of plasmid loss per 10 generations is 97% in pHARS1 transformants, the predominant part of the Ura$^+$ cells after 100 generations should be integrants. The Ura$^+$ colonies tested were all shown to maintain a stable Ura$^+$ phenotype indicating an integration of the URA3 gene. This was further verified by Southern blot analysis. In addition, these data indicate that the integration frequency is $5 \times 10^{-6}$.

Example 1

CLONING OF THE GENE FOR ALCOHOL OXIDASE (MOX) FROM HANSENULA POLYMORPHA

Characterization of polyadenylated RNA

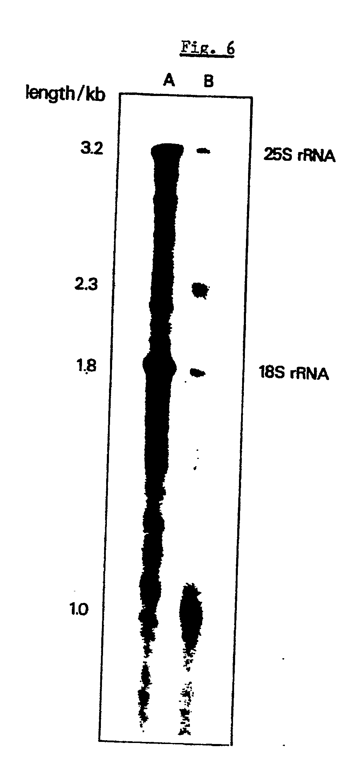

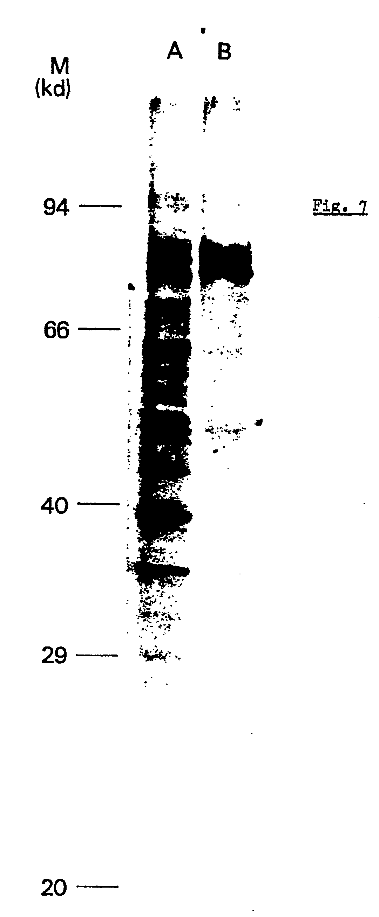

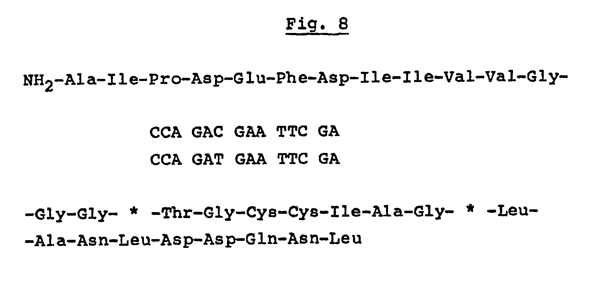

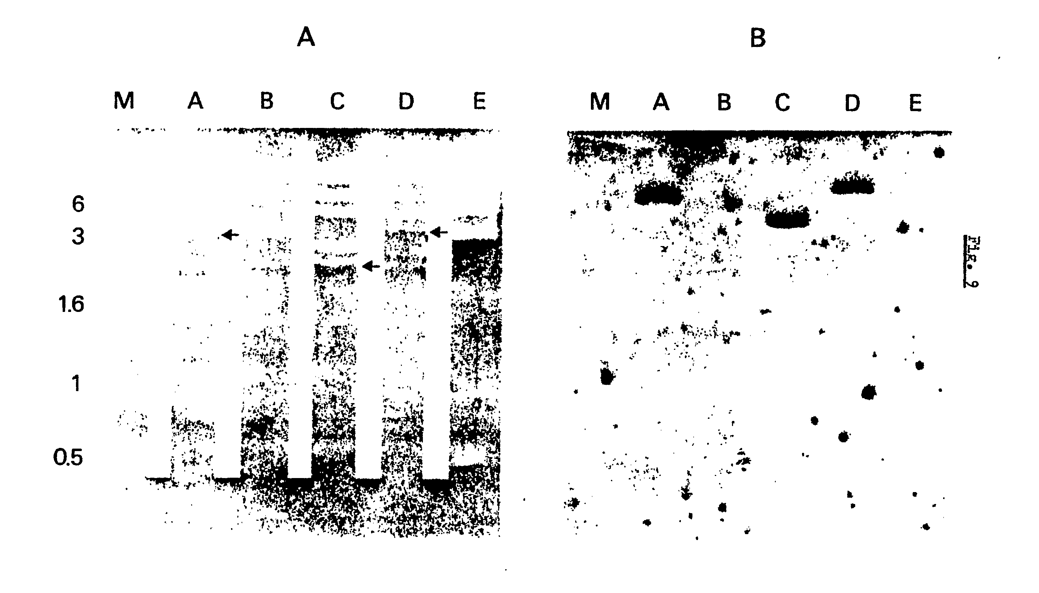

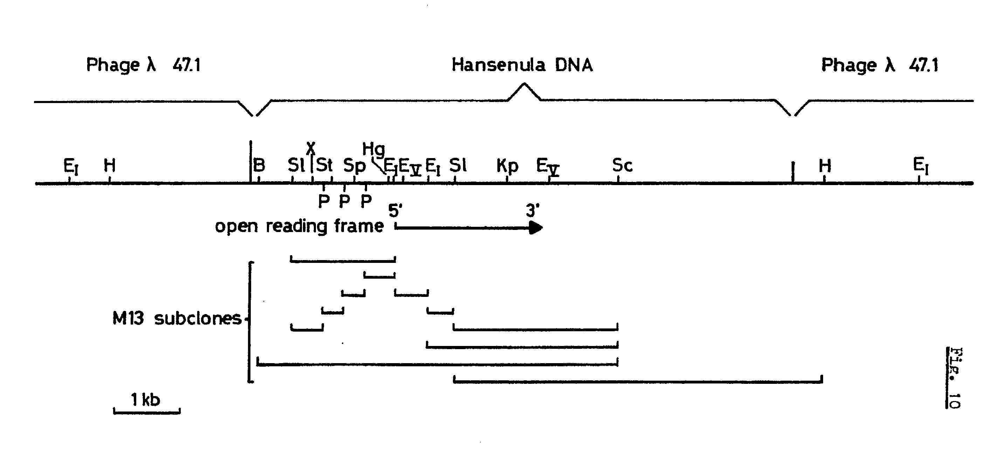

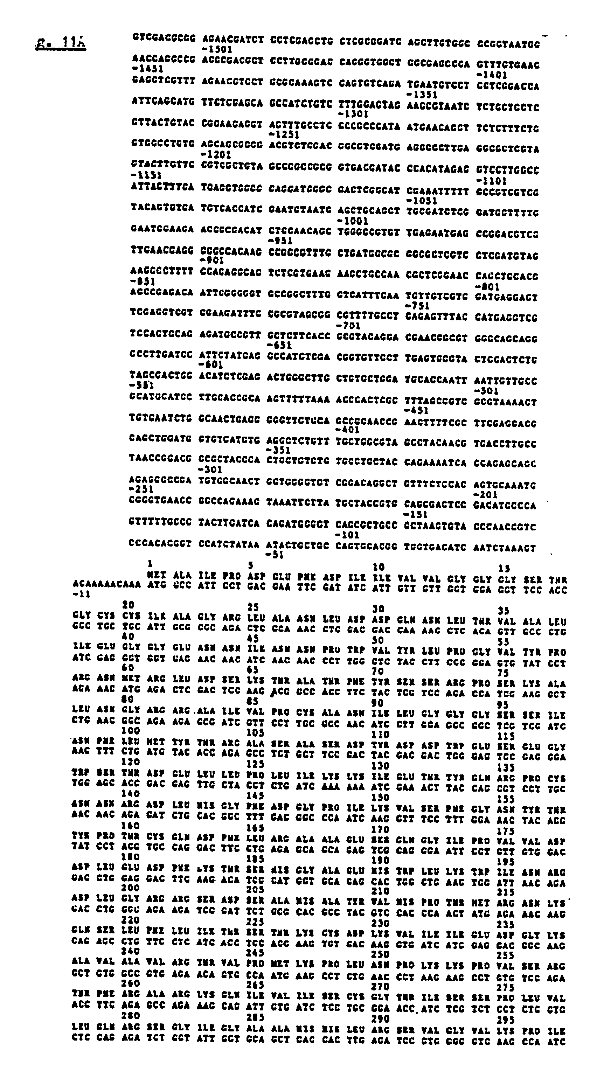

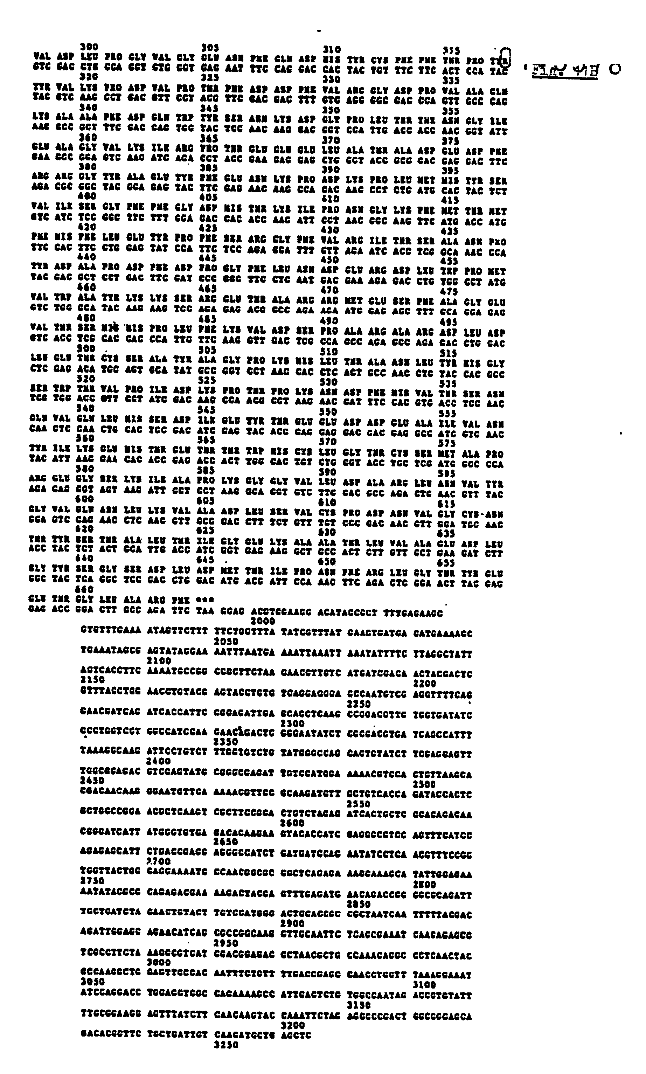

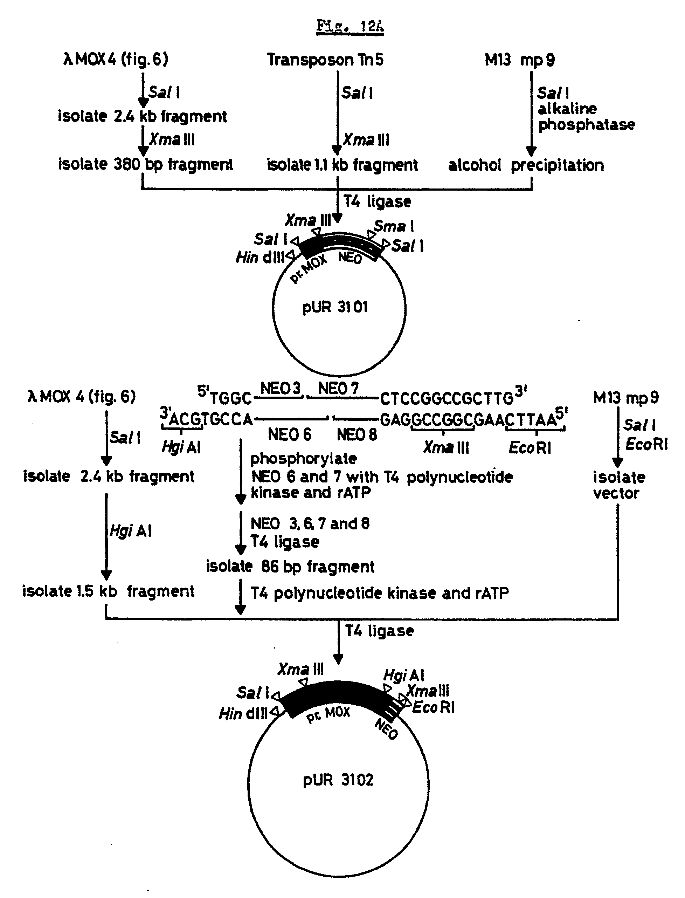

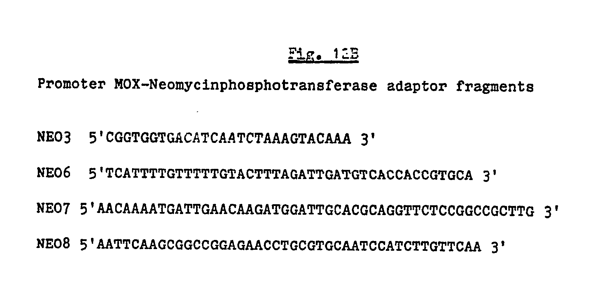

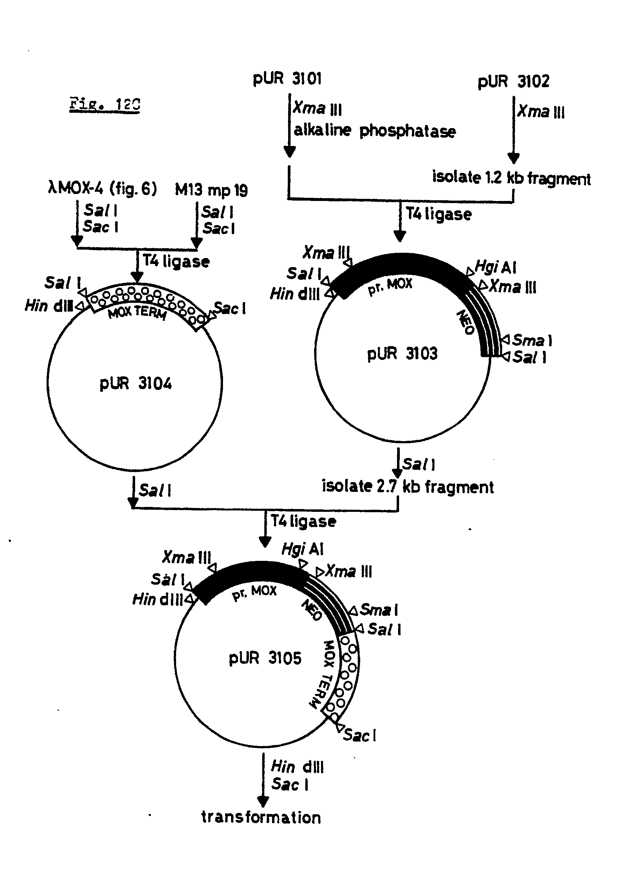

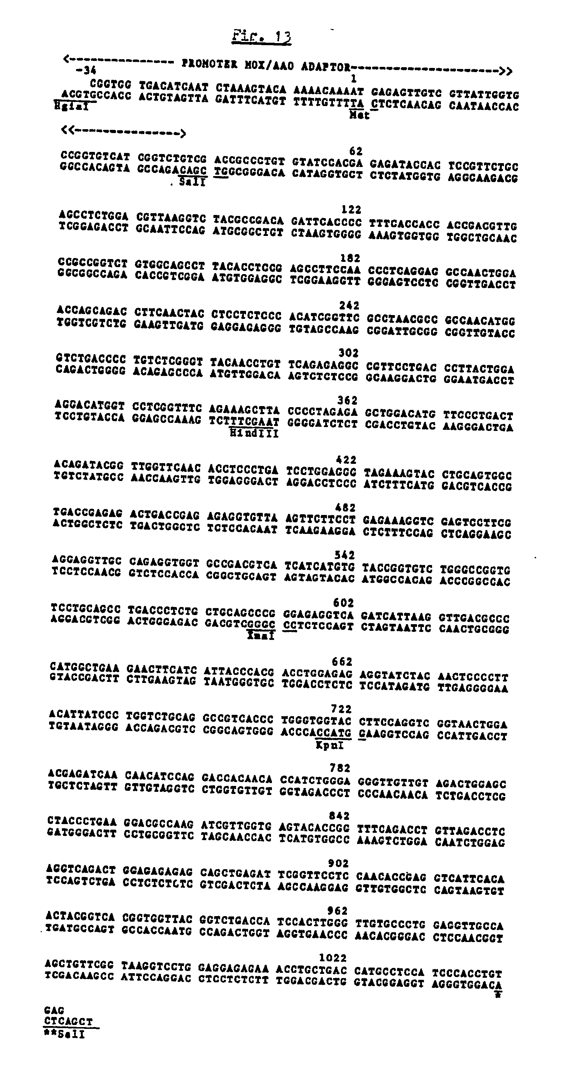

The total RNA and polyadenylated RNA, isolated from cells grown on methanol, were labelled at their 3′-termini with ATP:RNA adenyl transferase, and separated on a denaturing polyacrylamide gel (Fig. 6). Apart from the rRNA bands, two classes of RNA appear in the poly-adenylated RNA lane, respectively 1 kb and 2.3 kb in length. Since these RNA classes are not found in polyadenylated RNA of ethanol-grown cells (result not shown), they obviously are transcripts of genes derepressed by growth on methanol. The 2.3 kb class can code for a protein of 700 to 800 amino acids, depending on the length of the non-translated sequences. Likewise, the 1 kb class codes for a protein of 250-300 amino acids. Enzymes that are derepressed by growth on methanol and are 700 to 800 amino acids long, most likely are MOX (Kato et al., 1976; Roa and Blobel, 1983) and DHAS (Bystrykh et al., 1981). Derepressed enzymes in the 250 to 300 amino acid range are probably formaldehyde and formate dehydrogenase (Schütte et al., 1976). The polyadenylated RNA was characterized further by in vitro translation in a reticulocyte cell free translation system. Two microliters of the polyadenylated RNA directed protein mixture was separated directly on a 10% SDS polyacrylamide gel, while the remaining 18 microliters were subjected to immuno-precipitation with antiserum against MOX (Fig. 7). Six strong bands dominate in the total protein mixture, having molecular weight of respectively 78kd, 74kd, 58kd, 42kd, 39kd and 36kd. Essentially the same molecular weights were found by Roa and Blobel (1983) in a total cell extract from methanol-grown H . polymorpha cells.

The 74kd protein can tentatively be assigned to the monomer of MOX, the 58kd protein to the monomer of catalase and the 39kd and 36kd proteins to the monomers of formaldehyde dehydrogenase and formate dehydrogenase, respectively. The 78kd polypeptide possibly is DHAS, while the 42kd polypeptide remains unidentified. After immuno-precipitation, both high molecular weight proteins react with the MOX antiserum.

Cloning of the gene for MOX

Although the 2.3 kb mRNA class induced by growth on methanol obviously codes for at least 2 polypeptides, it seemed a good candidate for screening a Hansenula polymorpha clone bank by hybridization. The 5-20 kb fraction of partially Sau 3AI digested H . polymorpha DNA was cloned in phage lambda L47.1.

Per microgram insert DNA, 300 000 plaques were obtained while the background was less than 1:1000. Two Benton Davis blots, containing about 20 000 plaques each, were hybridized with 15 000 cpm of the mRNA-derived cDNA probe. After 3 weeks of autoradiography about 40-50 hybridizing plaques could be detected. All plaques were picked up and five were purified further by plating at lower density and by a second hybridization with the cDNA probe. From four, single hybridizing plaques (1, 3, 4, 5) DNA was isolated. The insert length varied from 8 to 13 kb.

Hybridization selection using organic-synthetic DNA probes

The sequence of 30 amino acids at the amino terminus of purified MOX was determined (Fig. 8).

Using the most abundant codon use for the yeast S . cerevisiae , a sequence of 14 bases could be derived from part of this protein sequence, with only one ambiguity. Both probes, indicated in Fig. 4, were synthesised. In both probes an Eco RI site is present. DBM blots were made from the DNA of the MOX clones digested with the restriction enzymes Bam HI, Eco RI/Hin dIII, Hin dIII/Sal I and Pst I/Sal I and separated on 1.5% agarose gels. After hybridization of the blot with a mixture of both radioactively labelled probes, the clones 1 , 4 and 5 hybridize, while clone 3 does not, as shown for the Hin dIII/Sal I blot in Fig. 9. However, the probes did not hybridize with the Eco RI/Hin dIII digested DNA of these clones (result not shown). Since an Eco RI site is present in the probes, the hybridizing DNA in the clones probably is cut by this enzyme too. Consequently the hybridization overlap has become too small to allow the formation of stable hybrids.

Restriction map and sequence analysis

By comparing restriction enzyme digests and by cross-hybridization experiments it was concluded that clones 1, 4 and 5 covered identical stretches of DNA.

In order to definitely establish the nature of this stretch of cloned DNA the insert of clone 4 was analyzed in detail. Hybridization with the amino terminal probe showed that the complete MOX gene (ca. 2 kb) was present, including 2 kb sequences upstream and 3.5 kb downstream (Fig. 10).

DNA sequence analysis of the smallest Eco RI fragment revealed the nucleotide sequence corresponding to the amino terminus of MOX as was determined by amino acid sequence analysis.

For sequence analysis, several fragments were subcloned in M13mp8/M13mp9 or M13mp18/M13mp19 respectively in two orientations, as indicated in Fig. 10. Clones that were smaller than 0.5 kb were sequenced directly from both sides. The larger clones were cut at the unique restriction sites situated in the middle of the cloned fragment, to allow generation of exonuclease Bal 31 digested subclones as described in materials and methods. Using specific oligonucleotide primers, sequences around the restriction sites used for subcloning and sequences that did not allow an unequivocal sequence determination were sequenced once more, using the 5.5 kb Bam HI/Sac I subclone that covers the whole sequence. The complete nucleotide sequence is given in Fig. 11A and 11B.

The sequence contains an open reading frame of 2046 nucleotides that can code for a protein of 664 amino acids. The last codon of the open reading frame codes for Phe, which is in agreement with the carboxy terminus of purified MOX. The amino acid composition derived from the DNA sequence encoding this protein, and the amino acid composition of purified MOX are virtually identical (Table III). The only important differences involve the serine and threonine residues, which are notoriously difficult to determine.

The calculated molecular weight of the protein is 74 050 Dalton, which agrees well with the molecular weight of 74 kd of MOX, as determined on polyacrylamide/SDS gels.

Codon usage

In Table IV the codon usage for MOX is given. A bias towards the use of a selective number of codons

9

is evident.

Example 2

CONSTRUCTION OF A PLASMID, pUR 3105, BY WHICH THE GENE CODING FOR NEOMYCIN PHOSPHOTRANSFERASE, THAT CONFERS RESISTANCE AGAINST THE ANTIBIOTIC G 418, IS INTEGRATED INTO THE CHROMOSOMAL MOX GENE UNDER REGIE OF THE MOX REGULON.

H . polymorpha cells, transformed with either the plasmids YEP 13, YRP 17, pHARS1 or pHARS2, were unstable and lost their leu[+] or ura[+] phenotype already after 10 generations upon growth under non-selective conditions. In order to obtain stable transformants and to test the MOX promoter, a plasmid pUR 3105 is constructed in which the neomycin phosphotransferase gene (NEO[R]) is brought under direct control of the MOX regulon. The construction is made in such a way that the first ATG of the NEO[R] gene is coupled to 1.5 kb of the MOX regulon. The cloning of such a large regulon fragment is necessary as shorter fragments, that do not contain the -1000 region of the regulon, were less efficient.

The NEO[R] gene was isolated as a 1.1 kb Xma III-Sal I fragment from the transposon Tn5, situated from 35 bp downstream of the first ATG up to 240 bp downstream of the TGA translational stop codon. To avoid a complex ligation mixture, first pUR 3101 is constructed (Fig. 12A), which is a fusion of the far upstream Sal I-Xma III (position -1510 to position -1128) fragment of the MOX regulon, and the NEO[R] gene, subcloned on M13mp9. Another plasmid is constructed, pUR 3102, in which the 1.5 kb Sal I-HgiA I fragment of the MOX gene, that covers nearly the whole MOX regulon, is ligated to a MOX-NEO[R] adapter (Fig. 12B) sequence and cloned in M13-mp9. The 1.2 kb Xma III fragment of this plasmid is cloned in to the Xma III site of pUR 3101, resulting in pUR 3103, which is the exact fusion of the MOX regulon and the NEO[R] gene (Fig. 12C). The orientation is checked by cleavage with HgiA I and Sal I. From the lambda-MOX-4 clone, a Sal I-Sac I fragment is subcloned that reaches from the Sal I site, still in the structural MOX gene (position 894), up to the Sac I site, far downstream of the structural MOX gene (position 3259) (see Fig. 10). This M13mp19 subclone is called pUR 3104. The plasmid pUR 3105 is obtained by the direct ligation of the 2.7 kb Sal I fragment from pUR 3103 into the Sal I site of pUR 3104. The orientation is tested by cleavage with Sma I and Sac I.

After cleavage of this plasmid with Hin dIII and Sac I and the transformation of this cleaved plasmid to H . polymorpha , G 418-resistant colonies are found that do not lose their resistance upon growth under non-selective conditions for a large number of generations.

Example 3

THE CONSTRUCTION OF pUR 3004, BY WHICH THE GENE CODING FOR D-AMINO ACID OXIDASE IS TRANSFERRED TO THE CHROMOSOME OF H. POLYMORPHA UNDER REGIE OF THE MOX-REGULON

D-amino acid oxidase (AAO) is an example of an oxidoreductase for the production of which the methylotrophic H . polymorpha is extremely suited. It might be expected that the enzyme, being an oxidase like MOX, is translocated to the peroxisomes of the yeast that are induced during growth on methanol or a mixture of methanol and a fermentable sugar as carbon source and D-amino acids as the sole nitrogen source. Under these conditions the cell will be protected from the $H_2O_2$ produced. Alternatively, AAO can be produced without the production of $H_2O_2$, when it is placed under regie of the MOX- or DAS-regulon. The AAO production will be induced by the presence of methanol in the medium.

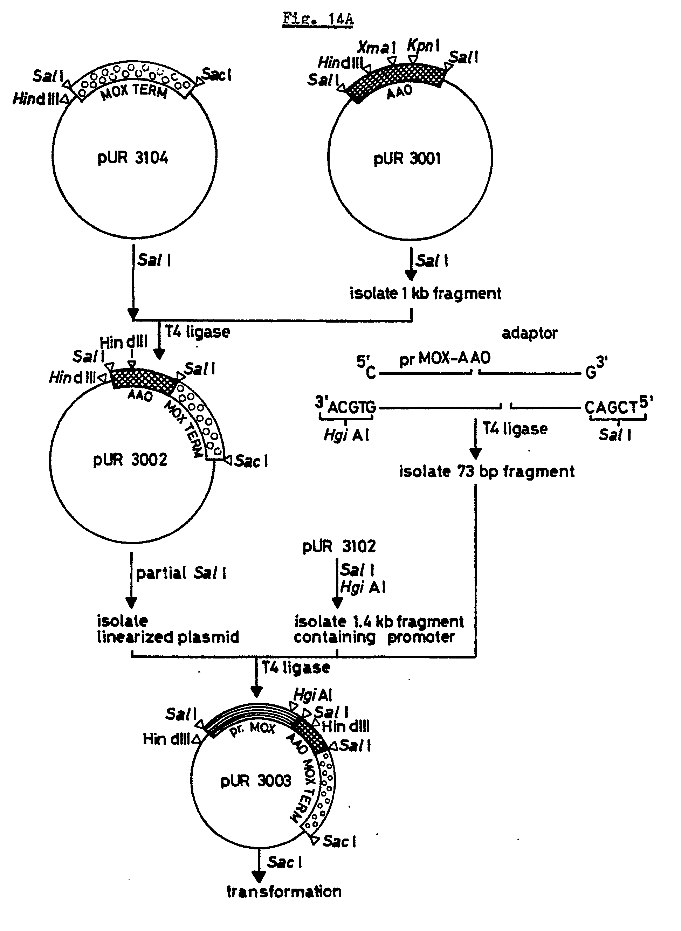

The amino acid sequence of the AAO enzyme has been published (Ronchi et al., 1981) and the complete gene is synthesised, using the phosphite technique (Matteuci and Caruthers, 1981). The gene is constructed in such a way that the optimal codon use for H . polymorpha , as derived from the sequence of the MOX gene, is used. Moreover, several unique restriction sites are introduced without changing the amino acid sequence, to facilitate subcloning during the synthesis. The DNA sequence is shown in Fig. 13. The gene is synthesised in oligonucleotides of about 50 nucleotides in length. Oligonucleotides are purified on 16% polyacrylamide gels. The oligonucleotides that form a subclone are added together in ligase buffer (Maniatis et al., 1982) and heated to 70°C in a waterbath. The waterbath is slowly cooled to 16°C and $T_4$-ligase is added. After two hours of ligation, the DNA is separated on a 1.5% agarose gel and the fragment, having the expected length, is isolated from the gel. It is subcloned in an m13mp18 vector cleaved at the respective restriction sites situated at the end of the fragment. The gene is subcloned in this way in 4 subclones, respectively Sal I-Hin dIII (position 39-346), Hin dIII-Xma I (position 346-589), Xma I-Kpn I (position 589-721) and Kpn I-Sal I (position 721-1044). The Sal I-Hin dIII and Hin dIII-Xma I subclones and the Xma I-Kpn I and Kpn -I-Sal I subclones are ligated together as two Sal I-Xma I subclones in Sal I-Xma I cleaved M13mp18. These two subclones are ligated into a Sal I cleaved M13mp8, resulting in pUR 3001 (Figs 13, 14A). The whole sequence is confirmed by the determination of the nucleotide sequence using the modified Sanger dideoxy sequencing technique (Biggin et al., 1983).

The construction of the integrative plasmid, containing the AAO gene is shown in Fig. 14A,B. The nearly complete AAO gene is placed upstream of the MOX termination region, by insertion of the AAO gene-containing Sal I fragment of pUR 3001, in the unique Sal I site of pUR 3104 (see also Fig. 14A), resulting in pUR 3002. The orientation is checked by cleavage with Hin dIII. The MOX promoter region is isolated as a 1.4 kb Sal I-HgiA I fragment from pUR 3102 (Fig. 14A). This fragment is subsequently placed upstream of the AAO gene in pUR 3002, by ligation to partially Sal I-digested pUR 3002 in the presence of the HgiA I-Sal I MOX-AAO adapter, shown in Fig. 14A. The orientation of the resulting plasmid pUR 3003 is checked again by cleavage with Hin dIII. This plasmid is integrated into the MOX gene after cleavage with Sac I and transformation to H . polymorpha cells. Transformants are selected by their ability to grow on D-amino acids as nitrogen source in the presence of methanol as inducer.

As the selection of cells containing the AAO gene is not simple, another selection marker is introduced. To this end, the S . cerevisiae LEU2 gene is integrated in between the structural AAO gene and the MOX terminater. For this construction, the plasmid pURS 528-03 is used. This plasmid is derived from pURY 528-03 described in European patent application 0096910. The construction is shown in Fig. 14C. The deleted carboxy terminal LEU2 gene sequence of pURY 528-03 was replaced by the complete carboxy terminal LEU2 gene sequence from pYeleu 10 (Ratzkin and Carbon, 1977) and the E . coli lac-lac regulon was eliminated. Subsequently the Hpa I-Sal I fragment of pURS 528-03 containing the LEU2 gene, is blunt end inserted in the Sal I site of pUR 3003, situated in between the AAO structural gene and the MOX terminater. The orientation of the resulting plasmid pUR 3004 can be checked by cleavage with Sal I and Sac I. pUR 3004 integrates in the chromosomal MOX gene of H . polymorpha after transformation of the Sac I-cleaved plasmid to a H . polymorpha leu⁻ mutant. Selected leu⁺ transformants are integrated in the chromosomal MOX gene, together with the AAO gene.

## Example 4

THE CONSTRUCTION OF pUR 3204, pUR 3205, pUR 3210 and pUR 3211, BY WHICH THE SMALL PEPTIDE HORMONE, THE HUMAN GROWTH RELEASING FACTOR, IS EXPRESSED UNDER REGIE OF THE MOX-REGULON, EITHER BY INTEGRATION INTO THE CHROMOSOMAL MOX GENE (pUR 3203, pUR 3204), OR BY INTEGRATION INTO A HARS1-CONTAINING PLASMID (pUR 3205) OR BY FUSION TO THE MOX STRUCTURAL GENE (pUR 3209, pUR 3210 and pUR 3211).

Human growth hormone releasing factor (HGRF) is a small, 44 amino acids long, peptide, that activates the secretion of human growth hormone from the pituitary glands. HGRF can be used in the diagnosis and treatment of pituitary dwarfism in man. Since HGRF has been shown to induce growth hormone stimulation in numerous species, HGRF might be used in the vetinary field too, by stimulating growth of animals and increase of milk production (Coudé et al., 1984). It is difficult to obtain HGRF from human sources, but it could very well be produced by biotechnological processes, once the gene has been cloned and transferred to an appropriate host organism. Also, as a general example of the production of a peptide hormone by H . polymorpha , the gene for HGRF is synthesised in the optimal codon use of H . polymorpha and brought to expression in several ways.

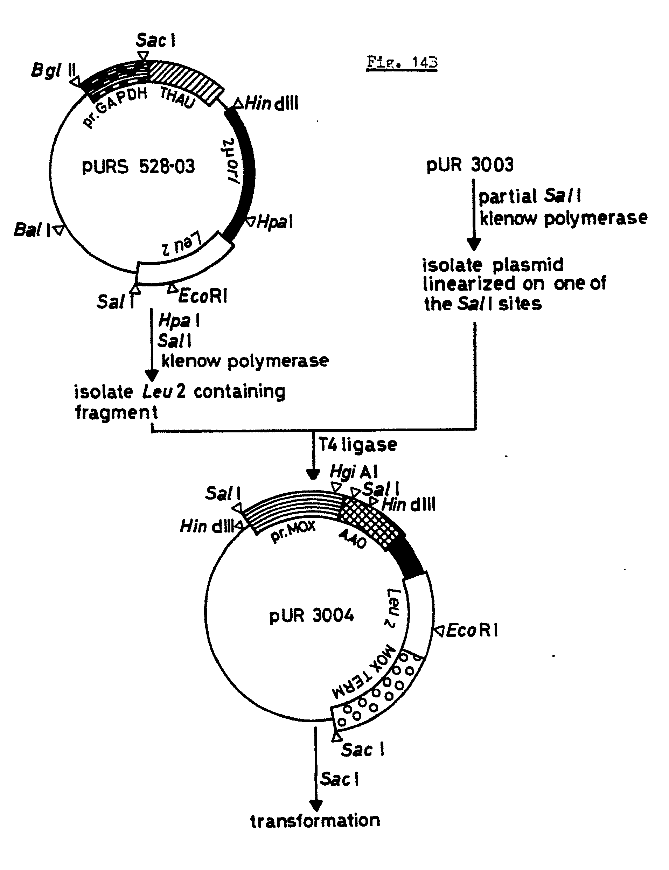

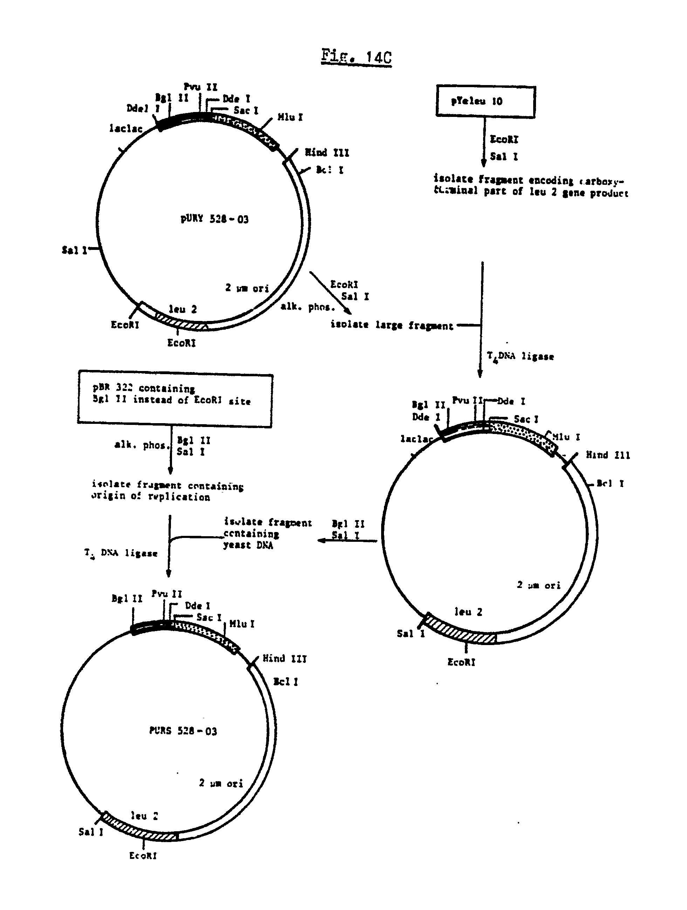

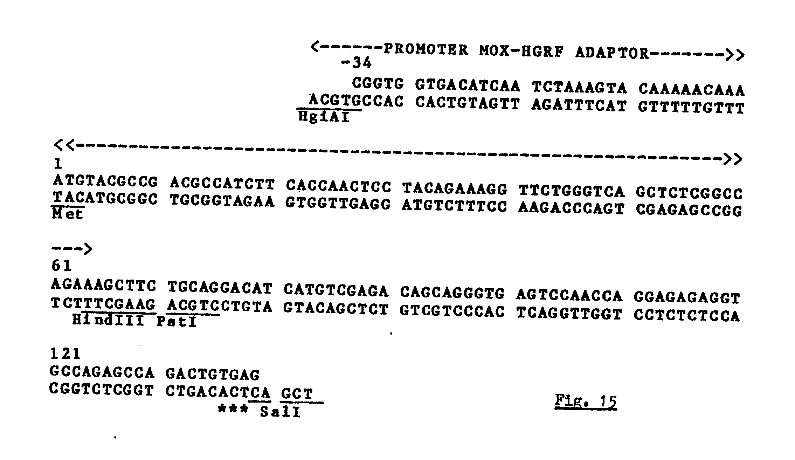

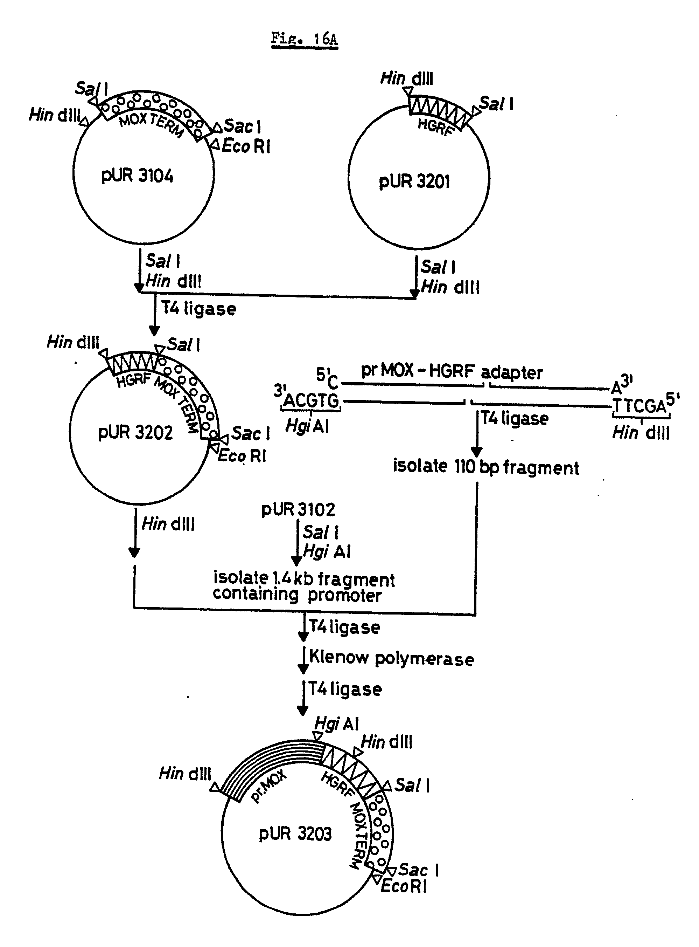

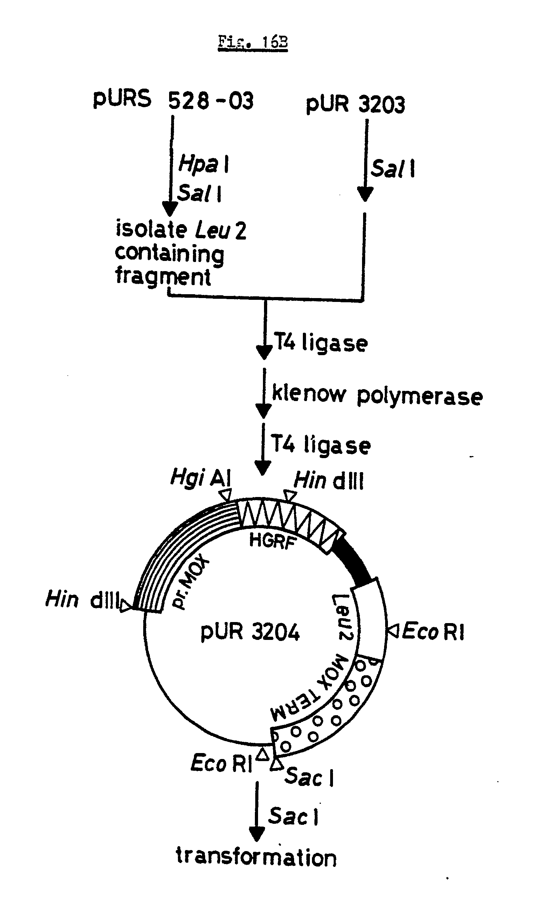

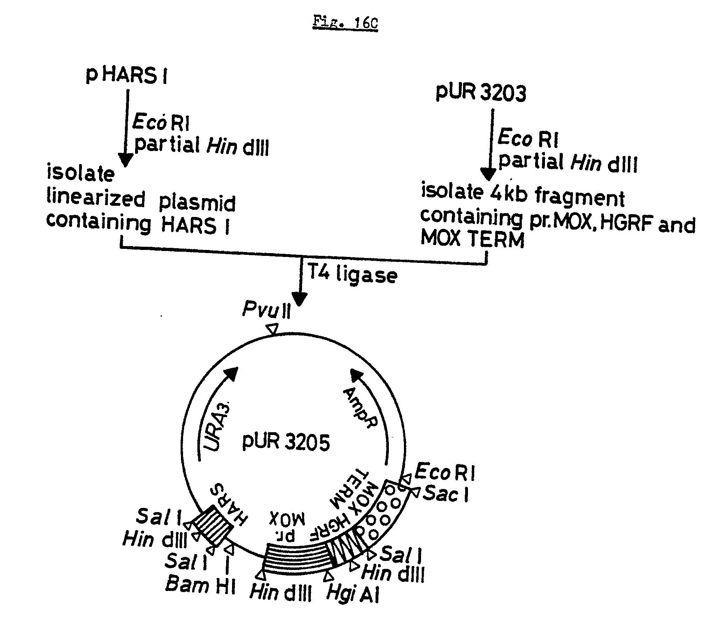

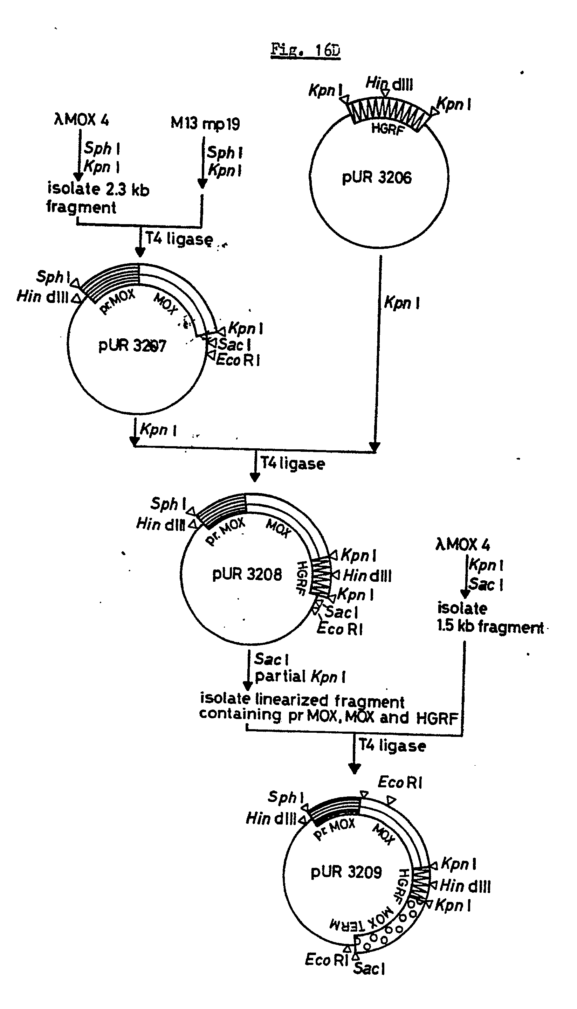

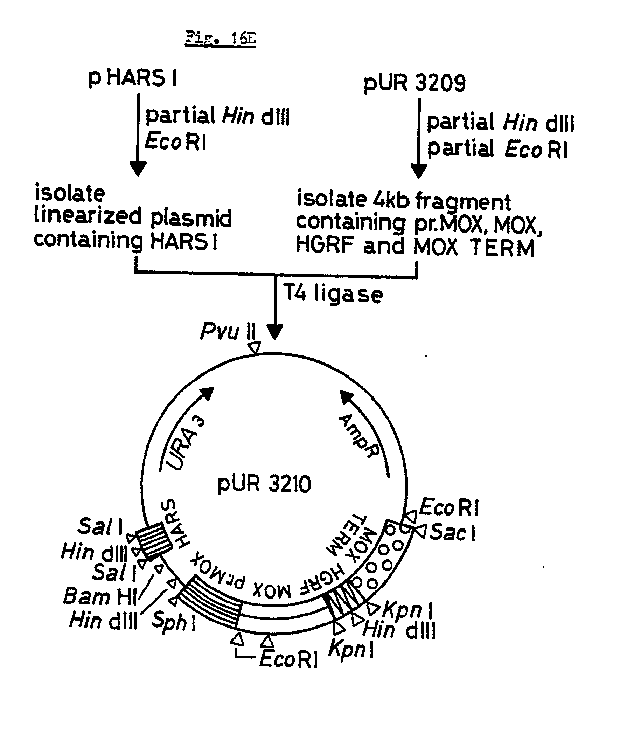

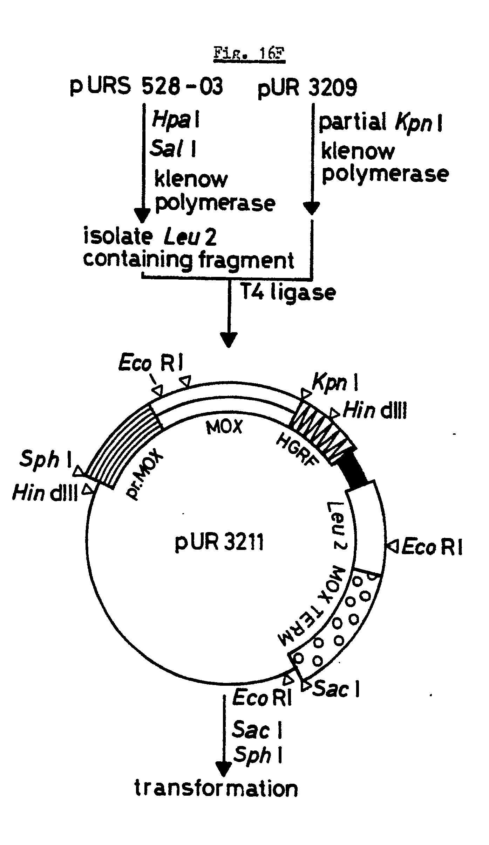

For the construction of pUR 3204 and pUR3205, the gene fragment that codes for the carboxy terminal part of the protein is synthesised in DNA oligomers of about 50 nucleotides in length and subcloned as a Hin dIII-Sal I fragment in Hin dIII-Sal I cleaved M13mp18, resulting in pUR 3201 (Figs. 15, 16A). This Hin dIII-Sal I fragment is subsequently inserted upstream of the MOX terminater in Hin dIII-Sal I cleaved pUR 3104 (Fig. 16A), resulting in pUR 3202. The MOX promoter is inserted in front of the HGRF gene, by insertion of the Sal I-Hgi AI MOX-promoter fragment from pUR 3102 (Fig. 16A) in Hin dIII cleaved pUR 3202, using a Hgi AI-Hin dIII adapter between the MOX-promoter and the HGRF gene (Figs 15, 16A). The orientation of the resulting plasmid pUR 3203 is checked by cleavage with Sal I and Hgi AI. pUR 3203

integrates into the chromosomal MOX gene of H . polymorpha after transformation of the Sac I cleaved plasmid. Transformants are selected on immunological activity. pUR 3203 is cleaved with Sal I, to insert the Sal I-Hpa I fragment of pURS 528-03 (Fig. 16B) that contains the LEU2 gene. The orientation of this gene in pUR 3204 is checked by cleavage with Hin dIII and Eco RI. pUR 3204 integrates into the chromosomal MOX gene of H . polymorpha after transformation of the Sac I cleaved plasmid (Fig. 16B) to a leu⁻ H . polymorpha mutant. Selection on on leu transformants. A plasmid, called pUR 3205, that replicates autonomously in H . polymorpha and contains the HGRF gene, is obtained by insertion of the Eco RI, partially Hin dIII cleaved 4 kb long fragment of pUR 3203, containing the HGRF gene inserted in between the MOX-promoter and terminater, into partially Hin dIII-Eco RI cleaved pHARS1 (Figs 2, 16C). The construction of pUR 3205 is checked by cleavage with Hin dIII.

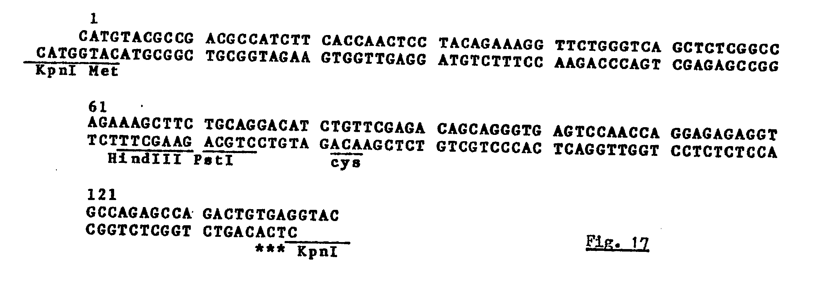

The production of small peptides as HGRF by microorganisms is often unstable as a result of enzymic degradation (Itakura et al., 1977). Fusion to a protein like MOX, and subsequent transport to the peroxisomes, could prevent degradation. Therefore, we decided to insert the HGRF gene into the unique Kpn I site at position 1775 (amino acid 591, Figs 10, 11) of the MOX structural gene. The HGRF gene is synthesised again in DNA oligomers of 50 nucleotides in length, but now as two Kpn I-Hin dIII subclones that are cloned as a complete HGRF structural gene in M13mp19, cleaved with Kpn I (plasmid pUR 3206, Figs 17, 16D). Moreover, the ATG triplet coding for the internal methionine of HGRF at position 27 (Coudé et al., 1984) (position 82 of the DNA sequence) is converted into a TGT triplet coding for cysteine. This does not alter the HGRF activity essentially, and facilitates the cleavage of HGRF from the fusion protein by CNBr cleavage (Itakura et al., 1977). From phage lambda MOX-4 (Fig. 10 Sph I (position -491)-Kpn I fragment is isolated and in serted into Sph I-Kpn I cleaved M13mp19. This results in pUR 3207. pUR 3206 is cleaved with Kpn I and the HGRF gene is inserted into the Kpn I site of pUR 3207, resulting in pUR 3208. The orientation is checked by direct sequence analysis on the single-stranded DNA of pUR 3208. Subsequently the downstream part of the MOX gene, from the unique Kpn I site up to the Sac I site, is isolated as a 1.5 kb fragment from phage lambda MOX-4 and inserted into Sac I - partially Kpn I cleaved pUR 3208. The orientation of the resulting plasmid pUR 3209 is checked by digestion with Kpn I. pUR 3209 integrates into the chromosomal MOX gene of H . polymorpha after transformation of the Sac I, Sph I cleaved plasmid. Selection on immunological activity.

This MOX-HGRF fusion gene is inserted into pHARS1 by isolation of the whole fusion gene from partially Hin dIII, partially Eco RI cleaved pUR 3209, into Eco RI partially Hin dIII cleaved pHARS1. This results in pUR 3210, which replicates in H . polymorpha after transformation (Fig. 16E). Alternatively, the LEU2-containing Sal I-Hpa I fragment of pURS 528-03 is inserted into the blunt-ended Kpn I site of the HGRF gene, located at the carboxy terminus of the encoded protein, after partial Kpn I cleavage of pUR 3209. The resulting plasmid pUR 3211 integrates into the chromosomal MOX gene of H . polymorpha , after transformation of the Sac I, Sph I cleaved plasmid (Fig. 16F).

## Discussion

From the length of the open reading frame, from the similarity in the amino acid composition of purified MOX and the DNA derived protein sequence and from the identical 30 N-terminal amino acids, it is concluded that the complete gene for MOX from the yeast Hansenula polymorpha has been cloned. Its calculated molecular weight agrees well with the molecular weight determined on SDS polyacrylamide gels. Apart from the coding sequence, more than 1200 bp has been sequenced from both the 5'- and the 3'-non-coding regions, reaching from the Sal I site upstream of the coding sequence, up to the Sac I site downstream. The gene appears not to be interrupted with intervening sequences.

The protein is not transcribed in the form of a precursor. Based on the determination of the molecular weight, N-terminal signal sequences could not be detected in earlier studies of Roa and Blobel (1983) or Roggenkamp et al. (1984) as well. In similar studies, it was suggested that also the rat liver peroxisomal enzymes uricase (Goldman and Blobel, 1978) and catalase (Goldman and Blobel, 1978; Robbi and Lazarow, 1978) do not contain a cleavable N-terminal signal peptide. However, as discussed by these authors, proteolytic degradation could possibly explain the lack of the detection of such a signal sequence.

Our sequence results definitely prove that for translocation of this protein to the peroxisome, a cleavable N-terminal signal sequence is not required. Such a translocation signal may well be situated in the internal sequence of the mature protein, as is the case for ovalbumine (Lingappa et al., 1979). Inspection of the protein sequence reveals the amino acid sequence Gly X Gly Y Z Gly (amino acids 13-18), which is charac teristic for FAD-(flavin adenine dinucleotide)-containing enzymes (Ronchi et al., 1981).

The isolation of the MOX gene described above gives a way how to determine the DNA sequence coding for MOX and the amino acid sequence of the MOX enzyme.

Similarly, the DNA sequences and amino acid sequences belonging to other oxidase-enzymes can be isolated and determined. The knowledge of the MOX gene sequence can be used to facilitate the isolation of genes coding for alcohol oxidases or even other oxidases. By comparing the properties and the structure of enzymes one can probably establish structure function and activity relationships. One can also apply methods as site-directed mutagenesis, or shortening or lengthening of the protein coding sequences, modifying the corresponding polypeptides, to select oxidase-enzymes with improved properties, e.g. with increased alkali stability, improved production, or oxidase-enzymes which need a substrate which is more compatible with detergent products.

Besides the isolation and characterization of the structural gene for MOX from the yeast H . polymorpha , also the isolation and characterization of the structural gene for DHAS from the yeast H . polymorpha has been carried out in a similar way.

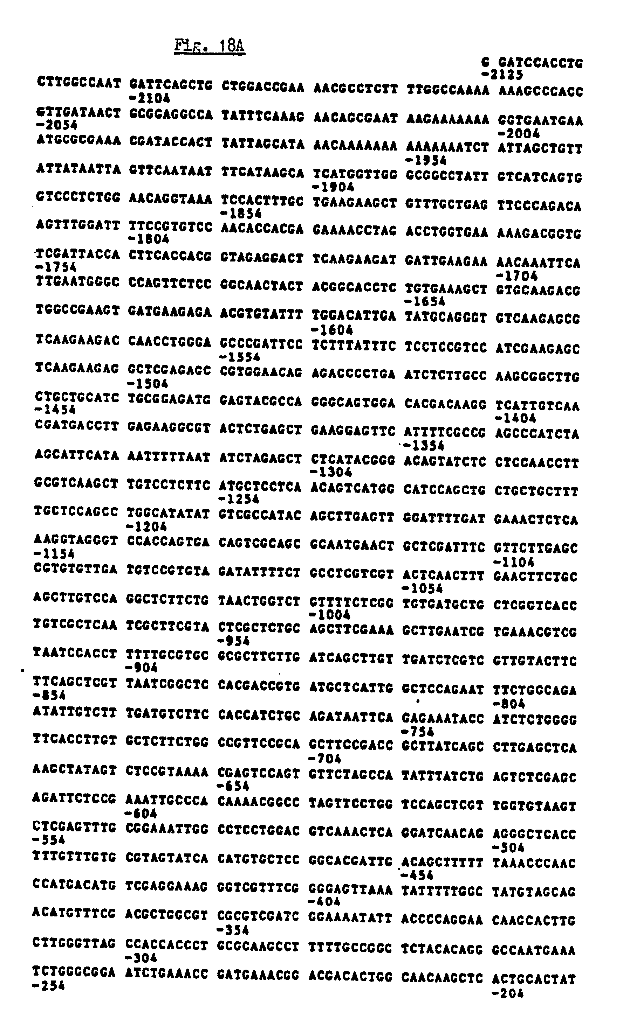

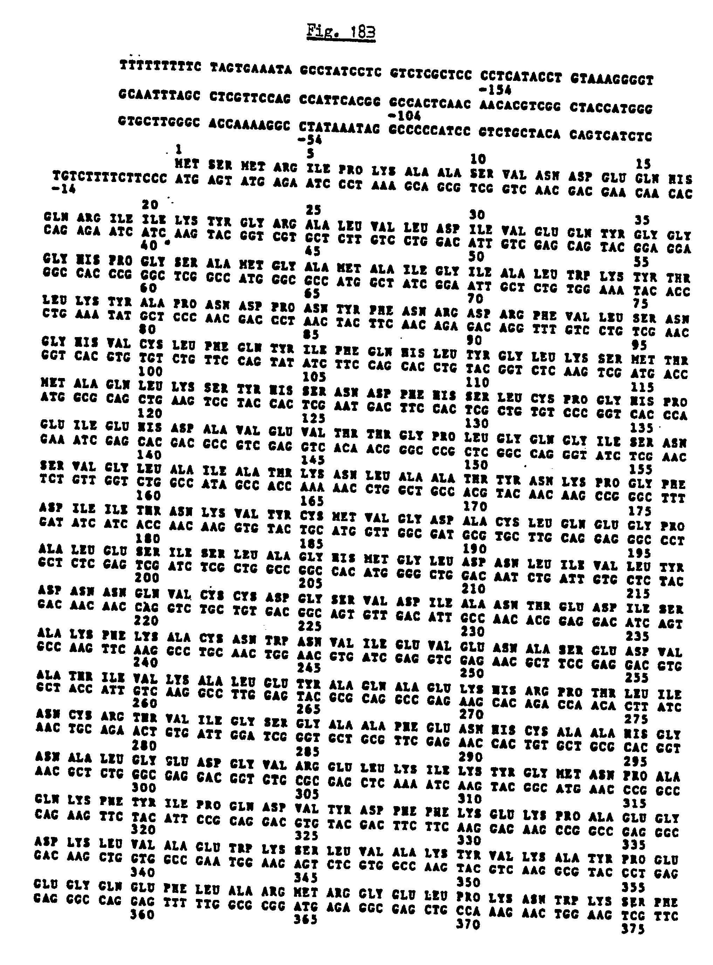

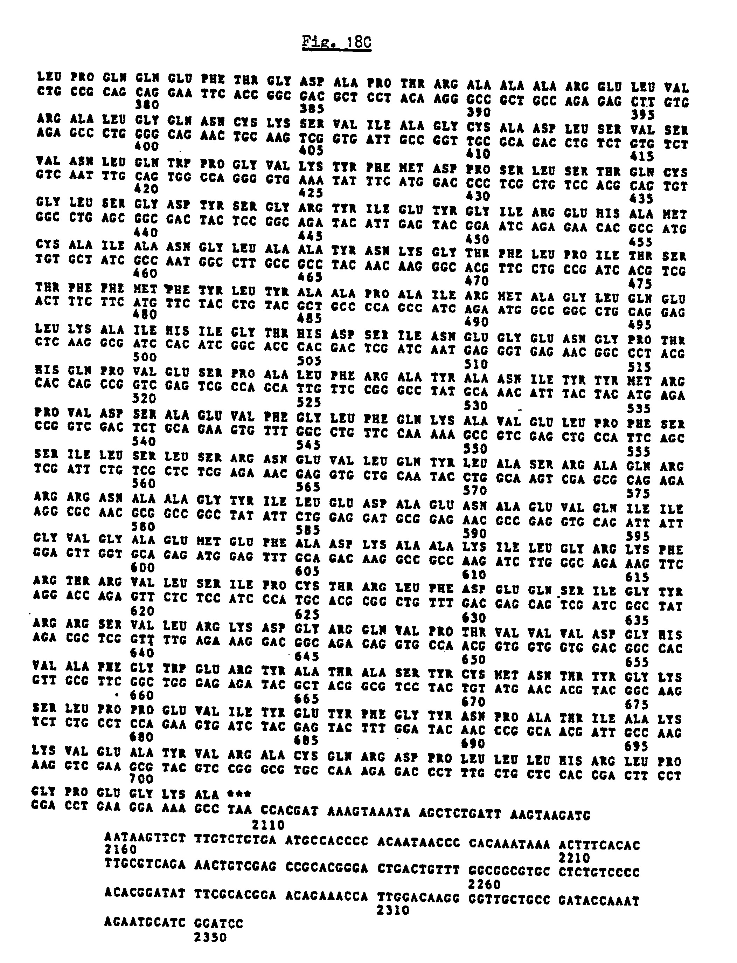

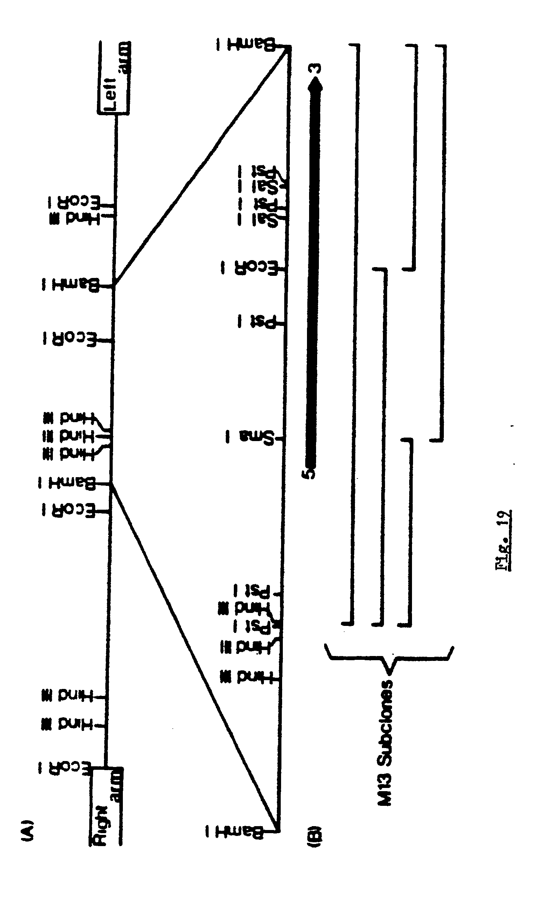

The DNA sequence of DAS is given in Fig. 18A-18C. A restriction map is given in Fig. 19. The amino acid composition calculated from the DNA sequence of DAS appeared to be in agreement with the amino acid composition determined after hydrolysis of purified DHAS. The DHAS enzyme catalyses the synthesis of dihydroxyacetone from formaldehyde and xylulose monophosphate. This reaction plays a crucial role in the methanol- assimilation process (cf. Veenhuis et al., 1983).

As described before, the synthesis of MOX and DHAS is subject to glucose repression. It has now been found that higher levels of MOX are reached when using glucose/methanol mixtures as substrates instead of 0.5% (v/v) methanol. Under the former conditions up to 30% of the cellular protein consists of MOX, compared with up to 20% under the latter conditions.

It was considered that in the regulons of MOX and DAS sequences must exist that play a decisive role in the regulation of repression/derepression by glucose or of the induction by methanol. Some homology therefore might be expected.

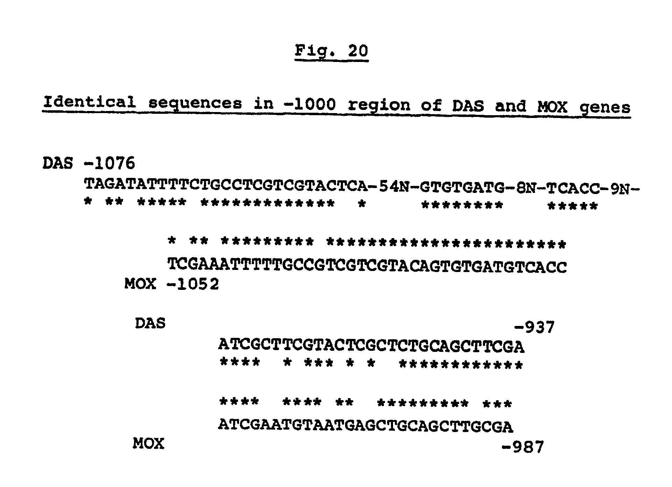

A striking homology of the "TATA-boxes" has been found, both having the sequence CTATAAATA. No other homologies in the near upstream region of the MOX and DAS regulons have been found. Unexpectedly, a detailed study of both regulons has shown a remarkable homology of the regulons for MOX and DAS in the region about 1000 bp upstream of the translation initiation codon. A practically complete consecutive region of 65 bp in the regulon of MOX is homologous to a 139 bp region in the DAS regulon, interspersed by several non-homologous regions (see Fig. 20). A similar homology is not found in any other region of both genes, that are over 4 kb in length including their upstream and downstream sequences. It is suggested that these homologous sequences play a role in the regulation of both genes by glucose and methanol. Transformation studies with vectors containing as regulon the first 500 bp upstream of the ATG of the structural gene of MOX, showed that this shortened MOX-regulon gave rise to a relatively low expression of the indicator gene beta-lactamase. Indicator genes are genes which provide the yeast with properties that can be scored easily, e.g. the gene for neomycin phosphotransferase giving resistance to the antibiotic G 418 (cf. Watson et al., 1983) or an auxotrophic marker such as leucin.

The fact that the far upstream homologous regions in the MOX and DAS genes have different interruptions and the fact that DAS is repressed at 0.1% glucose and MOX is not, suggest that these homologous regions are of importance to the repression-derepression by glucose and/or the induction of the expression in the presence of methanol. This assumption has been found correct indeed, and the presence or absence of these homologous regions can therefore be important for specific applications. For example, if the -1052 to -987 region of the MOX gene or the -1076 to -937 region of the DAS gene is important for the induction of MOX or DAS by methanol, the presence of these regions is required for the expression of MOX or DAS and/or for the induction of other enzymes by methanol. Another example might be the removal of the regions to avoid repression by glucose, which is needed for the expression of genes coding for proteins other than MOX and DHAS under influence of the MOX and/or DAS regulatory regions with glucose as a carbon source.

Thus one aspect of the present invention relates to the isolation and complete characterization of the structural genes coding for MOX and DHAS from the yeast H . polymorpha . It further relates to the isolation and complete characterization of the DNA sequences that regulate the biosynthesis of MOX and DHAS in H . polymorpha , notably the regulons and terminaters.

Moreover, it relates to combinations of genes coding for alcohol oxidase or other oxidases originating from H . polymorpha strains other than H . polymorpha CBS 4732, or Hansenula species other than H . polymorpha , or yeast genera other than Hansenula , or moulds, or higher eukaryotes, with the powerful regulon and terminater of the MOX gene from H . polymorpha CBS 4732. These combinations may be located on vectors carrying amongst others an autonomously replicating sequence originating from H . polymorpha or related species or minichromosomes containing centromers, and optionally selection marker-

(s) and telomers. These combinations may also be integrated in the chromosomal DNA of H . polymorpha .

Furthermore it relates to combinations of the powerful regulon or parts of it and terminaters of the MOX and/or DAS and - by site-directed mutagenesis or other methods - changed structural genes coding for alcohol oxidase or another oxidase. These changed structural genes may be located on episomal vectors, in mini-chromosomes or integrated in the chromosomes of H . polymorpha , H . wingeii , H . anomala , and S . cerevisiae or in other yeasts.

The parent application EP-PA 85201235.0 published as EP-A2- 0 173 378 relates to combinations of the regulon and terminater of the MOX and/or DAS gene of H . polymorpha with structural genes coding for other proteins than oxidases.

A very important and preferred embodiment of that invention is a process for preparing a polypeptide, such as a protein or an enzyme, by culturing the microorganism under suitable conditions, optionally concentrating the polypeptide and collecting same in a manner known per se , characterized in that a microorganism is used that has been obtained by recombinant DNA technology and caries a structural gene coding for the polypeptide concerned, the expression of which is under the control of a regulon, comprising a promoter and at least either the -1052 to -987 region of the MOX gene of Hansenula polymorpha CBS 4732, or the -1076 to -937 region of the DAS gene of Hansenula polymorpha CBS 4732, or a corresponding region of other methylotrophic moulds or yeasts, or an effective modification of any of these regions.

Surprisingly, it has been observed by the present inventors that the regions concerned, which are shown in Fig. 20 and are referred to herein as the -1000 regions of the MOX and DAS genes, are of crucial importance for the expression of the structural gene concerned. Experiments performed with recombinants containing the MOX regulon from which this region was eliminated showed a low level of expression. Therefore, use of a regulon comprising such -1000 region, or an effective modification thereof, i.e. any modification which does not result in a significant mutilation of the function of said region, makes it possible to realize production of a relatively high amount of the desired polypeptide.

A preferred embodiment of the process according to that invention is characterized in that the structural gene concerned has been provided with one or more DNA sequences coding for amino acid sequences involved in the translocation of the gene product into the peroxisomes or equivalent microbodies of the microbial host. Translocation of the produced polypeptide into the peroxisomes or equivalent microbodies improves their stability, which results in a higher yield. For certain kinds of polypeptides, in particular oxidases, ** such translocation is imperative for survival of the microbial host, i.e. to protect the host against the toxic effects of the hydrogen peroxide produced when the microbial host cells are growing on the substrate of the oxidase. If the oxidase concerned does not contain addressing signals which are functional in the microbial host used in the production process, one should provide the structural gene with sequences coding for host specific addressing signals, for example by adding such sequences or by substituting these for the original addressing sequences of the gene. Production of a fused polypeptide, in which the fusion partner carries suitable addressing signals, is another possibility. In case methylotrophic yeasts are used in the production process, it is preferred that the DNA sequences consist of the MOX gene or thos parts thereof which are responsible for MOX translocation into the peroxisomes or microbodies.

Finally, this aspect of the present invention is related to the synthesis of MOX originating form H . polymorpha in other yeasts.

Some microorganisms with the potential of producing alcohol oxidases are summarized below.

Yeasts producing alcohol oxidases (Taxonomic division according to Lee and komagata, 1980)

Group 1 Candida boidinii
Group 2a Hansenula philodendra
Pichia lindnerii
Torulopsis nemodendra
Torulopsis pinus
Torulopsis sonorensis
Group 2b Candida cariosilignicola
Hansenula glucozyma
Hansenula henricii
Hansenula minuta
Hansenula nonfermentans

** thus also in the present invention,

Hansenula polymorpha
Hansenula wickerhamii
Pichia pinus
Pichia trehalophila
Group 2c Candida succiphila
Torulopsis nitratophila
Group 3 Pichia cellobiosa
Group 4 Hansenula capsulata
Pichia pastoris
Torulopsis molischiana
Moulds producing alcohol oxidases:
Lenzites trabea
Polyporus versicolor
Polyporus obtusus
Poria contigua

Among the oxidases other than alcohol oxidases, the most interesting are:
- glycerol oxidase,
- aldehyde oxidase,
- amine oxidase,
- aryl-alcohol oxidase,
- amino acid oxidase,
- glucose oxidase,
- galactose oxidase,
- sorbose oxidase,
- uric acid oxidase,
- chloroperoxidase, and
- xanthine oxidase.

Combinations of the powerful regulons and terminaters of the MOX and DAS genes from H . polymorpha and structural genes for oxidases may be combined with one or more DNA sequences that enable replication of the structural gene in a particular host organism or group of host organisms, for example autonomously replicating sequences or centromers (and telomers) originating from H . polymorpha , to suitable vectors that may be transferred into H . polymorpha and related yeasts or other microorganisms.

H . polymorpha mutants LEU-1 and LR9, mentioned on page 12 of this specification, were deposited at the Centraalbureau voor Schimmelcultures at Delft on 15th July, 1985, under numbers CBS 7171 and CBS 7172, respectively.

The above description is followed by a list of references, claims, Tables, Legends to Figures and Figures.

References

1. GB-PS 1 225 713 (Colgate-Palmolive Company; publ. 24th March 1971; priority date 19th April 1968).
2. DE-PA 2 557 623 (Henkel & Cie GmbH; publ. 30th June 1977; priority date 20th December 1975).
3. GB-PA 2 101 167 (Unilever PLC; publ. 12th January 1983; priority date 7th July 1981).
4. van Dijken, J.P., Otto, R. and Harder, W. (1976), Arch.Microb. 111 , 137-144.
5. Veenhuis, M., van Dijken, J.P. and Harder, W. (1983) in Advances in Microbial Physiology, Rose, A.H., Gareth Morris, J. and Tempest, D.W., Eds, Vol. 24, pp 1-82, Academic Press, New York.
6. Roggenkamp, R., Janowicz, Z., Stanikowski, B. and Hollenberg, C.P. (1984), Mol.Gen.Genet. 194 , 489-493.
7. Sahm, H. (1977) in Advances in Microbiol. Engineering, Ghose, T.K., Fiechter, A. and Blakebrough, N., Eds, Vol. 6, pp 77-103, Springer-Verlag, Berlin.
8. Bystrykh, L.V., Sokolov, A.P. and Trotsenko, Y.A. (1981), FEBS Letters 132 , 324-328.
9. Roa, M. and Blobel, G. (1983), Proc.Natl.Acad.Sci. USA, 80 , 6872-6876.
10. Veenhuis, M., van Dijken, J.P., Pilon, S.A.F. and Harder, W. (1978), Arch.Microbiol. 117 , 1953-163.
11. Loenen, W.A.M. and Brammar, W.J. (1980), Gene 20 , 249-259.
12. Hohn, B. (1979) in Methods in Enzymology, Wu, R., Ed., Vol. 68, pp 299-309, Academic Press, New York.

13. Edens, L., Heslinga, L., Klok, R., Ledeboer, A.M., Maat, J., Toonen, M.Y., Visser, C. and Verrips, C.T. (1982), Gene 18 , 1-12.

14. Pelham, H.R.B. and Jackson, R.J. (1976), Eur.J. Biochem. 67 , 247-257.

15. Valerio, D., Duyvensteijn, M.G.C., Meera Khan, P., Geurts van Kessel, A., de Waard, A. and van der Eb, A.J. (1983), Gene 25 , 231-240.

16. Ledeboer, A.M., Verrips, C.T. and Dekker, B.M.M. (1984), Gene 30 , 23-32.

17. Maniatis, T., Fritsch, E.F. and Sambrook, J. (1982), Molecular Cloning, p 278, Cold Spring Harbor Laboratory Publ., New York.

18. Benton, W.D. and Davis, R.W. (1977), Science 196 , 180-182.

19. Ambler, R.P. (1972), Methods in Enzymology, Vol. 25, pp 262-272, Academic Press, New York.

20. Matteucci, M.D. and Caruthers, M.H. (1981), J.Am. Chem.Soc. 103 , 3185-3191.

21. Wallace, R.B., Johnson, M.J., Hirose, T., Miyake, T., Kawashima, E.H. and Itakura, K. (1981), Nucl. Acids Res. 9 , 879-894.

22. Biggin, M.D., Gibson, T.J. and Hong, G.F. (1983), Proc.Natl.Acad.Sci.USA 80 , 3963-3965.

23. Gleeson, M.A., Waites, M.J. and Sudbery, P.E. (1984), in: Microbial growth on $C_1$ compounds, Eds. Crawford, R.L. and Hanson, R.S., Publ. A.S.M., Washington, 228-235.

24. Fink, G.D. (1970), Methods in Enzymology, Tabor, H. and Tabor, C.W., Eds., Vol. 17, pp 59-78, Academic Press, New York.

25. Boeke, J.D., LaCroute, F. and Fink, G.D. (1984), Mol. Gen.Genet. 197 , 345-346.

26. Jones, E.W. and Fink, G.D. (1982), Cold Spring Harbour Monogr.Ser., 11B , 181-299.

27. Lieberman, I., Kornberg, A. and Simms, E.S. (1955), J.Biol.Chem. 215 , 403-415.

28. Stinchchomb, D.T., Thomas, M., Kelly, J., Selker, E. and Davis, R.W. (1980), Proc.Natl.Acad.Sci.USA 77 , 4559-4563.

29. Stinchcomb, D.T., Mann, C. and Davis, R.W. (1982), J.Mol.Biol. 158 . 157-179.

30. Struhl, K., Stinchcomb, D.T., Scherer, S. and Davis, R.W. (1979), Proc.Natl.Acad.Sci.USA 76 . 1035-1039.

31. Carlson, M. and Botstein, D. (1982), Cell 28 , 145-154.

32. Beggs, J.D. (1978), Nature 275 , 104-109.

33. Broach, J.R., Strathern, J.N. and Hicks, J.B. (1979), Gene 8 , 121-133.

34. Das, S., Kellerman, E. and Hollenberg, C.P. (1984), J.Bacteriol. 158 , 1165-1167.

35. Ito, H., Fukuda, Y., Murata, K. and Kimura, A. (1983), J.Bacteriol. 153 , 163-168.

36. Klebe, R.J., Harriss, J.V., Sharp, Z.D. and Douglas, M.G. (1983), Gene 25 , 333-341.

37. Hollenberg, C.P. (1982), Curr.Top.Microbiol.Immunol. 96 , 119-144.

38. Kato, N., Omori, Y., Tani, Y. and Ogata, K. (1976), Eur.J.Biochem. 65 , 341-350.

39. Schütte, H., Flossdorf, J., Sahm, H. and Kula, M.R., (1976), Eur.J.Biochem. 62 , 151-160.

40. Ronchi, S., Minchiotti, L., Galliano, M., Curti, B., Swenson, R.P., Williams, C.H. and Massey, V. (1981), J.Biol.Chem. 257 , 8824-8830.

41. Ratzkin, B. and Carbon, J. (1977), Proc.Natl.Acad. Sci.USA 74 , 487-491.

42. Coudé, F.X., Diaz, J., Morre, M., Roskam, W. and Roncucci, R. (1984), Trends in Biotechnology 2 , 83-88.

43. Itakura, K., Hirose, T., Crea, R., Riggs, A.D., Heyneker, H.L., Bolivar, F. and Boyer, H.W. (1977), Science 198 . 1056-1063.

44. Goldman, B.M. and Blobel, G. (1978), Proc.Natl. Acad.Sci.USA 75 , 5066-5070.

45. Robbi, M. and Lazarow, P.B. (1978), Proc.Natl.Acad. Sci.USA 75 , 4344-4348.

46. Lingappa, V.R., Lingappa, J.R. and Blobel, G. (1979), Nature 281 , 117-121.

47. Watson, J.D., Tooze, J. and Kurtz, D.T. (1983), Recombinant DNA, A Short Course, page 178, published by W.H. Freeman and Company, New York.

48. Lee, J.D. and Komagata, K. (1980), J.Gen.Appl. Microbiol. 26 , 133-158.

TABLE I

| Activities of orotidine 5′-phosphate decarboxylase and orotidine 5′-phosphate pyrophosphorylase in H . polymorpha mutants requiring uracil for growth. | | | |
|---|---|---|---|
| Strain/Genotype | Reversion rate | Activity (%)[a] | |
| | | Orotidine 5′-phosphate decarboxylase | Orotidine 5′-phosphate pyrophosphorylase |
| Wild type | - | 100 | 100 |
| LR 9/odcl | $< 2 \times 10^9$ | <1 | 106 |
| MR 7/odcl | $6 \times 10^7$ | <1 | 71 |
| NM 8/odcl | $3 \times 10^8$ | <1 | 105 |
| CLK 55/oppl | n.e.[b] | 90 | < 1 |
| CLK 68/oppl | n.e. | 82 | < 1 |
| YNN 27/ura3 | n.e. | 0 | n.e. |
| Strains were grown in YEPD until late exponential phase. Extraction of cells was performed with glass beads using a Braun homogenizer. Protein was estimated by the optical density at 280 nm. | | | |

[a] Expressed as the percentage of wild type activity.
[b] Not estimated.

TABLE II

| Transformation of uracil-requiring mutants of H . polymorpha | | | | |
|---|---|---|---|---|
| Strain | Plasmid | Transformation frequency[a] | Stability[b] (%) | Status of transformed DNA |
| LR 9 | YRP17 | $2.2 \times 10^2$ | <1 | Autonomous replication |
| LR 9 | pHARS1 | $1.5 \times 10^3$ | 2 | Autonomous replication |
| LR 9 | pHARS2 | $4.6 \times 10^2$ | 1.5 | Autonomous replication |
| LR 9 | YIP5 | 3 (38)[c] | 105 | Integration |
| LR 9 | pRB58 | 0 | - | - |
| LR 9 | pHH85 | 0 | - | - |
| YNN 27 | YIP5 | 0 | - | - |

[a] Expressed as total number per μg of DNA. Intact cells treated with polyethyleneglycol were used for transformation as described in Materials and Methods.
[b] Expressed as the percentage of remaining uracil prototrophs after growth on YEPD for ten generations.
[c] Number in parentheses indicates the amount of mini-colonies containing free plasmid YIP5.

TABLE III

| Amino acid composition of MOX | | |
|---|---|---|
| Amino Acid | DNA sequence | Hydrolysate [a] |
| PHE | 31 | 32 |
| LEU | 47 | 49 |
| ILE | 34 | 34 |
| MET | 12 | 11 |
| VAL | 42 | 43 |
| SER | 43 | 33 [a] |
| PRO | 43 | 42 |
| THR | 44 | 38 |
| ALA | 47 | 50 |
| TYR | 27 | 27 |
| HIS | 19 | 21 |
| GLN | 13 | |
| GLU | 36 | ] 51 |
| ASN | 32 | |
| ASP | 50 | ] 84 |
| LYS | 35 | 38 |
| CYS | 13 | 12 |
| TRP | 10 | - [b] |
| ARG | 36 | 36 |
| GLY | 50 | 53 |

a) Hydrolysis was performed for 24 h.
b) Not determined.

TABLE IV

| Comparison of preferred codon usage in S . cerevisiae , H . polymorpha and E . coli | | |
|---|---|---|
| Saccharomyces | Hansenula | E . coli |
| | MOX | |
| ALA GCU, GCC | GCC | GCC not used, no clear pref. |
| SER UCU, UCC | UCC, UCG | UCU, UCC |
| THR ACU, ACC | ACC | ACU, ACC |
| VAL GUU, GUC | GUA not used, no clear pref. | GUU, GUA |
| ILE AUU, AUC | AUC, AUU | AUC |
| ASP GAC | GAC | GAC |
| PHE UUC | UUC | UUC |
| TYR UAC | UAC | UAC |
| CYS UGU | no clear pref. | no clear pref. |
| ASN AAC | AAC | AAC |
| HIS CAC | CAC | CAC |
| GLU GAA | GAG | GAA |
| GLY GGU | GGC practically not used, no clear pref. | GGU, GGC |
| GLN CAA | CAG | CAG |
| LYS AAG | AAG | AAA |
| PRO CCA | CCU, CCA | CCG |
| LEU UUG | CUG, CUC | CUG |
| ARG AGA | AGA | CGU |

18

Legends to Figures

Fig. 1. The exonuclease Bal 31 digestion strategy used in sequencing specific MOX subclones. The fragment X-Y subcloned in M13mp-8 or -9, -18 or -19 is cut at the unique restriction site Z. The DNA molecule is subjected to a time-dependent exonuclease Bal 31 digestion. The DNA fragment situated near the M13 sequencing primer is removed using restriction enzyme Y; ends are made blunt end by incubation with $T_4$-DNA polymerase and then ligated intramolecularly. Phage plaques are picked up after transformation and the fragment is sequenced from site Z in the direction of site X. Using the M13 derivative with a reversed multiple cloning site, the fragment is sequenced from site Z in the direction of site X.

Fig. 2. Alignment of pHARS plasmids derived by insertion of HARS fragments into the single Sal I site of YIp5.

Fig. 3. The complete nucleotide sequence of the HARS-1 fragment.

Fig. 4. Estimation of copy number by Southern hybridization of H . polymorpha transformants. An aliquot of 8 and 16 $\mu$l of each probe was electrophoresed. Lane 1, phage lambda DNA digested with Hin dIII and Eco RI. Lanes 2,3 transformant of K . lactis containing two copies of integrated plasmid, digested with Hin dIII (M. Reynen, K. Breunig and C.P. Hollenberg, unpublished); lanes 4-7, YNN 27, transformed with pRB58 (4-5) and YRP17 (6-7) digested with Eco RI respectively; lanes 8,9, LR9 transformed with YRP17 digested with Eco RI; lanes 10,11, LR9 transformed with pHARS2 digested with Hin dIII; lanes 12,13, LR9 transformed with pHARS1 digested with Eco RI.

Fig. 5. Autoradiogram of Southern blots of DNA from H . polymorpha mutant LR9 transformed by integration of plasmid YIp5. Lane 1, phage lambda DNA, digested both with Hin dIII and Eco RI; lane 2, pHARS-1, undigested; lanes 3-5 and lanes 6,7 show DNA from 2 different transformants. Lane 3, undigested; lane 4, digested with Eco RI; lane 5, digested with Pvu II; lane 6, digested with Eco RI; lane 7, digested with Pvu II; lane 8, plasmid YIp5, digested with Eco RI. Nick-translated YIp5 was used as a hybridization probe.

Fig. 6 Electrophoresis of $^{32}$p-labelled RNA from Hansenula polymorpha , purified once (lane A) or twice (lane B) on oligo(dT)cellulose. Electrophoresis was performed on a denaturing 7 M urea 2.5% polyacrylamide gel. The position of the yeast rRNA's and their respective molecular weights are indicated by 18S and 25S. The 2.3 kb band, that can be seen in lane B, was converted into a cDNA probe which was subsequently used to isolate MOX and DHAS clones from the Hansenula polymorpha clone bank.

Fig. 7 $^{35}$S-labelled proteins obtained after in vitro translation of methanol derepressed, Hansenula polymorpha mRNA with a rabbit reticulocyte lysate. Either 2 microliters of the total lysate (lane A) or an immuno-precipitate of the remaining 18 microliters using a MOX specific antiserum (lane B) were separated on an 11.5% SDS-polyacrylamide gel. A mixture of proteins with known molecular weights was used as markers.

Fig. 8. The N-terminal sequences of purified MOX, as determined on a Beckman sequenator. The two probes that could be derived from the sequence Pro-Asp-Gln-Phe-Asp, using Saccharomyces preferred codons, are indicated.

Fig. 9. Hybridization of a DBM blot of Hin dIII/Sal I cut MOX clones. The DNA was separated on a 1.5% agarose gel (Fig. 9A) and the blot was hybridized to a mixture of both MOX-derived synthetic DNA probes (Fig. 8). Only one band of clones 1, 4 and 5 hybridize (Fig. 9B), indicated by an arrow in Fig. 9A. Lane M: molecular weight markers as indicated. Lane A, B, C and D: clones 1, 3, 4 and 5, respectively. Lane E: lambda L47.1.

Fig. 10. Restriction map for MOX clone 4. Only relevant restriction sites are indicated that have been used for subcloning and sequencing of the MOX gene. The open reading frame, containing the structural MOX sequence, and the M13 subclones made are depicted.Restriction sites used are: B = Bam HI, $E_I$ = Eco RI, $E_V$ Eco RV, P = Pst I, Sl = Sal I, Sc = Sac I, St = Stu I, H = Hin dIII, Sp = Sph I, K = Kpn I, Hg = Hgi AI and X = Xma I.

Fig. 11A,B. The nucleotide sequence of the MOX structural gene and its 5′- and 3′-flanking sequence.

Fig. 12A,C. The construction of plasmid pUR 3105 by which the neomycin phosphotransferase gene integrates into the chromosomal MOX gene of H . polymorpha .

Fig. 12B. Promoter MOX-neomycin phosphotransferase adapter fragments.

Fig. 13. The DNA sequence of the AAO gene, derived from the published amino acid sequence. The gene is synthesised in the optimal codon use for H . polymorpha in oligonucleotides of about 50 nucleotides long. Restriction sites, used for subcloning are indicated. The Hgi Al-Sal I fragment forms the adapter between the structural AAO gene and the MOX promoter. The translational start codon (met) and stop codon (***) are indicated. The structural sequence is numbered from 1 to 1044, while the MOX promoter is numbered from -34 to -1.

Fig. 14A. The construction of pUR 3003, by which the AAO gene integrates into the chromosomal MOX gene of H . polymorpha . Selection on activity of the AAO gene.

Fig. 14B. The construction of pUR 3004, by which the AAO gene integrates into the chromosomal MOX gene of a H . polymorpha leu⁻ derivative. Selection on leu⁺.

Fig. 14C. The construction of pURS 528-03. Owing to the removal of the pCR1 sequence and the double lac UV5 promoter, this plasmid is about 2.2 kb shorter than pURY 528-03.

Fig. 15. The DNA sequence of the HGRF gene, derived from the published amino acid sequence. The gene is synthesised in the optimal codon use for H . polymorpha in oligonucleotides of about 50 nucleotides long. Hgi Al, Hin dlll and Sal I sites are used for subcloning. The Hgi Al-Hin dlll fragment forms the adapter between the structural HGRF gene and the MOX promoter. The translational start codon (met) and stop codon (***) are indicated. The structural sequence is numbered from 1 to 140, while the MOX promoter is numbered from -34 to -1.

Fig. 16A. The construction of pUR 3203, by which the gene coding for HGRF integrates into the chromosomal MOX gene of H . polymorpha . Selection on immunological activity of HGRF.

Fig. 16B. The construction of pUR 3204, by which the gene coding for HGRF integrates into the chromosomal MOX gene of a H . polymorpha leu⁻ derivative. Selection on leu⁺.

Fig. 16C. The construction of pUR 3205, by which the gene coding for HGRF is inserted into a HARS-1-containing plasmid, which replicates autonomously in H . polymorpha . Selection by transformation of a ura⁻ mutant.

Fig. 16D. The construction of pUR 3209, by which the gene coding for HGRF integrates into the chromosomal MOX gene of H . polymorpha , fused to the structural MOX gene. HGRF is cleaved from the fusion protein by CNBr cleavage. Selection on immunological activity of HGRF.

Fig. 16E. The construction of pUR 3210, by which the gene coding for HGRF is inserted into a HARS-1-containing plasmid, fused to the structural MOX gene. Selection as in Fig. 16C.

16F. The construction of pUR 3211, by which the gene coding for HGRF integrates into the chromosomal MOX gene of a H . polymorpha leu⁻ derivative, fused to the structural MOX gene. Selection on leu⁺.

Fig. 17. The DNA sequence of the HGRF gene, derived from the published amino acid sequence. The gene is synthesised as mentioned in Fig. 15, but constructed in such a way that it could be inserted into the unique Kpn I site of the structural MOX gene. Therefore it was equipped with Kpn I sites on both sides of the gene, and Kpn I-Hin dlll fragments were used for subcloning. Synthesis will be as a fusion product to the MOX enzyme. The internal met (ATG) at position 82 is converted into a cys (TGT). Translational start (met) and stop (***) codons are indicated.

Fig. 18A,B,C. The nucleotide sequence of the DAS structural gene and its 5′- and 3′-flanking sequence.

Fig. 19. Restriction map for the DAS-lambda clone. Only relevant restriction sites are indicated that have been used for subcloning and sequencing of the MOX gene. The open reading frame, containing the structural DAS sequence, and the M13 subclones made, are depicted.

Fig. 20. Identical sequences in -1000 region of DAS and MOX genes.


## Claims

1. Process for preparing an oxidoreductase by culturing a microorganism under suitable conditions, optionally concentrating the enzyme and collecting the concentrated enzyme in a manner known per se , characterized in that a microorganism is used that has been obtained by recombinant DNA technology, and which is capable of producing the oxidoreductase.

2. Process according to claim 1, characterized in that the microorganism is capable of producing at least one enzyme selected from the group consisting of

(1) alcohol oxidases,

(2) amine oxidases, including alkylamine oxidase and benzylamine oxidase,

(3) amino acid oxidases, including D-alanine oxidase, lysine oxidase,

(4) cholesterol oxidase,

(5) uric acid oxidase,

(6) xanthine oxidase,

(7) chloroperoxidase, and

(8) aldehyde oxidase.

3. Process according to claim 1 or 2, characterized in that the microorganism is a mould or yeast.

4. Process according to claim 3, characterized in that a mould or yeast is selected from the group consisting of the genera Aspergillus , Candida , Geotrichum , Hansenula , Lenzites , Nadsonia , Pichia , Poria , Polyporus , Saccharomyces , Sporobolomyces , Torulopsis , Trichospora and Zendera .

5. Process according to claim 4, characterized in that the mould or yeast is selected from the species Aspergillus japonicus , Aspergillus niger , Aspergillus oryzae , Candida boidinii , Hansenula anomala , Hansenula polymorpha , Hansenula wingeii , Kloeckera sp.2201 and Pichia pastoris .

6. Process according to any one of claims 1-5, characterized in that the microorganism is also capable of producing a dihydroxyacetone synthase enzyme, which promotes the formation of dihydroxyacetone from formaldehyde.

7. Use of an oxidoreductase prepared by a process as claimed in any one of claims 1-5 in an oxidation process.

8. Bleaching composition including a fabric-washing detergent composition or hard-surface-cleaning composition having bleach activity, characterized in that it contains an oxidoreductase prepared by a process as claimed in any one of claims 1-5 and a substrate for that oxidoreductase.

9. Microorganism, obtainable by recombinant DNA technology and being capable of producing an oxidoreductase suitable for use in a process as claimed in claims 1-5.

10. Microorganism, obtainable by recombinant DNA technology and being capable of producing a dihydroxyacetone synthase-enzyme suitable for use in a process according to claim 6, in addition to being capable of producing an oxidoreductase.

11. Process for preparing a transformed microorganism as claimed in claim 9, characterized in that a DNA sequence coding for an oxidoreductase together with one or more other DNA sequences which regulate the expression of the structural gene is introduced into the microorganism via an episomal vector or integration in the genome, such that the microorganism is capable of producing the oxidoreductase.

12. Process for preparing a transformed microorganism as claimed in claim 10, characterized in that a DNA coding for a dihydroxyacetone synthase-enzyme together with one or more other DNA sequences which regulate the expression of the structural gene is introduced into the microorganism via an episomal vector or integration in the genome, such that the microorganism is capable of producing the dihydroxyacetone synthase-enzyme (DHAS enzyme).

13. DNA sequence coding for an oxidoreductase, characterized in that it is obtainable by recombinant DNA technology from natural and/or cDNA and/or chemically synthesised DNA.

14. DNA sequence according to claim 13, characterized in that it codes for an alcohol oxidase.

15. DNA sequence according to claim 14, characterized in that it comprises the DNA sequence 1-1992 (MOX gene) given in Fig. 11A + 11B encoding the polypeptide 1-664 (MOX), the amino acid sequence of which is given in Fig. 11A + 11B.

16. Combination of DNA sequences comprising a structural gene coding for an oxidoreductase and one or more other DNA sequences which regulate the expression of the structural gene in a particular microorganism or group of microorganisms.

17. Combination of DNA sequences according to claim 16, characterized in that it comprises at least part of the upstream DNA sequence -1 to about -1500 given in Fig. 11A and/or at least part of the down stream DNA sequence 1993 to about 3260 given in Fig. 11B (regulatory regions of the MOX gene).

18. Combination of DNA sequences according to claim 17, characterized in that it comprises at least the polynucleotide -1052 to -987 of the upstream DNA sequence given in Fig. 11A.

19. Combination of DNA sequences according to claim 17, characterized in that it contains a modified MOX promoter sequence which is obtainable by deletion of at least polynucleotide -1052 to -987 given in Fig. 11A.

20. Combination of DNA sequences according to claim 16, characterized in that it comprises at least part of the upstream DNA sequence -1 to about -2125 given in Fig. 18A + 18B and/or at least part of the downstream DNA sequence 2107 to about 2350 given in Fig. 18C (regulatory regions of the DAS gene).

21. Combination of DNA sequences according to claim 20, characterized in that it comprises at least the polynucleotide -1076 to -937 of the upstream DNA sequence given in Fig. 18A.

22. Combination of DNA sequences according to claim 20, characterized in that it contains a modified DAS promoter sequence which is obtainable by deletion of at least polynucleotide -1076 to -937 given in Fig. 18A.

23. Combination of DNA sequences according to claim 16, characterized in that it comprises a structural gene coding for an oxidoreductase of a higher eukaryote, a mould, or a yeast.

EP 0 423 890 A2

24. Combination of DNA sequences according to claim 23, characterized in that it comprises a structural gene coding for an oxidoreductase of a yeast of the genus Hansenula , preferably of the species H . polymorpha .

25. Combination of DNA sequences according to claim 16, characterized in that the structural gene coding for an oxidoreductase encodes an alcohol oxidase.

26. Combination of DNA sequences according to claim 25, characterized in that the structural gene is the DNA sequence 1-1992 (MOX gene) given in Fig. 11A + 11B encoding the polypeptide 1-664 (MOX), the amino acid sequence of which is given in Fig. 11A + 11B.

27. Combination of DNA sequences according to claim 16, characterized in that it also contains a structural gene coding for DHAS.

28. Combination of DNA sequences according to claim 27, characterized in that it contains a structural gene coding for DHAS having the amino acid sequence as given in Fig. 18B + 18C.

29. Combination of DNA sequences according to any one of claims 16-28, characterized in that the DNA sequences have been modified, while retaining their coding function for an oxidoreductase or for their regulatory functions, by recombinant DNA technology.

30. Combination of DNA sequences according to any one of claims 16-29, characterized in that it contains one or more DNA sequences that enable stable inheritance of said combination in the progeny of any particcular host organism.

31. DNA sequence coding for a dihydroxyacetone synthase-enzyme, characterized in that it is obtainable by recombinant DNA technology from natural and/or cDNA and/or chemically synthesised DNA.

32. DNA sequence according to claim 31, characterized in that it comprises the DNA sequence 1-2106 (DAS gene) given in Fig. 18B + 18C encoding the polypeptide 1-702 (DHAS), the amino acid sequence which is given in Fig. 18B + 18C.

33. Combination of a DNA sequence coding for a dihydroxyacetone synthase-enzyme and one or more other DNA sequences which regulate the expression of the structural gene in a particular microorganism or group of microorganisms.

34. Combination of DNA sequences according to claim 33, characterized in that it comprises the DNA sequence according to claim 32 (DAS gene) and at least part of the upstream DNA sequence -1 to about -2125 given in Fig. 18A + 18B and/or at least part of the downstream DNA sequence 2107 to about 2350 given in Fig. 18C (regulatory regions of the DAS gene) and/or at least part of the upstream DNA sequence -1 to about -1500 given in Fig. 11A and/or at least part of the downstream DNA sequence 1993 to about 3260 given in Fig. 11B (regulatory regions of the MOX gene).

35. Combination of DNA sequences according to claim 34, characterized in that it comprises at least the polynucleotide -1076 to -937 of the upstream DNA sequence given in Fig. 18A or at least the polynucleotide -1052 to -987 of the upstream DNA sequence given in Fig. 11A, respectively.

22

Fig. 1

Fig. 2

Fig. 3 DNA sequence of the autonomous-replicating sequence HARS1
from the methylotrophic yeast Hansenula polymorpha. The
HARS1 represents a Sall fragment comprising 483 nucleo-
tides. The dideoxy-sequencing method was employed.

```
(GTCGACTCCG CGACTCGGCG TTCACTTTCG AGCTATTCAT CAACGCCGGA ATACGTCAGA
AACAGCCGTG CCCCAGGGAC CAGAAAGCCT ACTGGTGAGT ATGTTCTTTC GTGTGATTTT
CCGAGGATGA GACGACGATA ACGAGCACAA CTCGGAGTCG GAGGACACGC TTATTGCGTT
GACGAGCCAC ATCAGCAGGC TGTCAAGACT GAGTATAGGC CACAGAGCTG ATTCTGCTCA
TACTCAAGAC GTTAGTAAAC TCCGTCTGCC ACAATGCTGA CAGAGTATTA TAATAATAGT
GAATTACGAA CAATGTAGTC AAAAAAATTT AGTAACAATA TGTCATGATG ACAGATTTGC
TGAAACCAGT GAACTCCAAT AAATCCAGCG GCTACCGCAT CCCAAGAGAA ACAGATCAGA
GGTCTAGGCT TGTTTCAGAG TACTACAAGC TTTCCAGAAC TTAGCAATTC TCAAACGCGG
TTTG(CGAC)
     483
```

Fig. 4

Fig. 5

1   2 3 4 5 6 7  8

Fig. 6

Fig. 7

## Fig. 8

NH$_2$-Ala-Ile-Pro-Asp-Glu-Phe-Asp-Ile-Ile-Val-Val-Gly-

CCA GAC GAA TTC GA
CCA GAT GAA TTC GA

-Gly-Gly- * -Thr-Gly-Cys-Cys-Ile-Ala-Gly- * -Leu-
-Ala-Asn-Leu-Asp-Asp-Gln-Asn-Leu

Fig. 9

Fig. 10

F. 11A

```
GTCGACGCGG AGAACGATCT CCTCGACCTG CTCCGCGGATC AGCTTGTGCC CCGGTAATGG
-1501
AACCAGCCCG ACGCGACGGT CCTTGCGGAC CACGGTGGCT GGCGAGCCCA GTTTGTGAAC
-1451                                                           -1401
GAGGTCGTTT AGAAGGTCCT GCGGCAAAGTC GAGTGTCAGA TGAATGTCCT CCTCGGACCA
                                                            -1351
ATTCAGGCATG TTGTCGAGCA GCCATGTGTG TTTGGACTAG AAGCCGTAATC TCTGCTCCTC
                                                            -1301
CTTACTGTAC CGGAAGAGGT ACTTTGCCTG CCCGCCCATA ATCAACAGCT TGTGTTTGTG
            -1251
GTGGCCTGTG AGCAGCCGGG ACGTGTGGAC GGGGTGGGATC AGGGGCCTTGA GGGGCTGGTA
-1201                                                          -1101
GTAGTTGTTG CGTGGCTGTA GCCGGCCGCG GTGACGGATAC CCACATAGAG GTGCTTGGCC
-1151                                                          -1101
ATTACTTTGCA TGACCTGCCC CAGGATGGGC GACTGGGCATG CGAAATTTTT GCCGTGGTGG
                                                            -1051
TACAGTGTGA TGTGCACCATC CAATCTAATG AGCTGCAGGT TGCGATGTGG GATGGCTTTG
                                                            -1001
GAATGGAAGA ACCGCGACAT GTCCAACAGC TGGGCCGTGT TGAGAATGAG CCGGACGTGG
                                                            -951
TTGAACGGAGG GCGGGCCACAAG CCGGCGTTTG CTGATGGGCGCC GCCGCTGGTC CTGGATGTAG
                                                            -901
AAGGGCCTTTT CCAGAGGCAG TGTGCCTGAAG AAGCTGGGCCAA CGCTGGGAAC CAGGTGGCACG
-851                                                          -801
AGGGGGAGACA ATTGGGGGGGT GGGGGGGCTTTG GTGCATTTGCAA TGTTGTGCGTC GATGAGGAGT
                                                            -751
TGGAGGTGGGT GGAAGATTTGC GGGGTGTAGCGG GGTTTTGCCCT GAGAGTTTAG GATGAGGTGG
                                                            -701
TGGCACTGGGCAG AGATGCGGCTT GGGTGCTTGCACC GGGTGTGTAGGA CGAAGGGGGCGT GGGGCAGGGAGG
                                                            -651
GGGGTTGGATGGCC ATTGGTGTATGAG GGGCATGCTCGA GGGTGGTTGCCT TGAGTGGGCGTA GTGCCACTGCTG
                    -601
TAGGGCACTGGG ACATGCTGCGAG ACTGGGGGCTTGG CTGTGGCTGCCA TGGCACCAATT AATTGTTGGCC
-551                                                         -301
GCATGGCATGCC TTGCCACCGGCA AGTTTTTTAAA ACCCACTGGCGC TTTAGGCCGGTC GGGTAAAAACT
                                                            -451
TGTGAATGCTC GCAACTGGAGC GGGGTTGCTGCCA CCCCCCAACCG AACTTTTTCGC TTGGGAGGGGACC
                    -401
CAGCTGGATGG GTGTGCATGCTG AGGGCTGCTGTT TGCTGGGCCGTA GCCTACAACG TGGACCTTGGCC
                    -351
TAAACCGGACG GCCCTACCCCA CTGGCTGTCTG TGGCCTGGCTAC CAGAAAATGCA CCAGAGGCAGGC
                    -301
AGAGGGGGCCGA TGTGGCCAACT GGTGGGGGCTGT CGGGACAGGGCT GTTTCTCCAC AGTGGCAAAATG
-251                                                          -201
CGGGTGAACC GGGCCAGAAAG TAAAATTCTTA TGGCTACCGTG CAGGGCGACTCC GACATCCCCA
                                                            -151
GTTTTTGGGCCC TACTTGATGCA CAGATGGGGGCT CAGCGGCTGGCC GGCTAACTGGTA CCCAACCGTC
                    -101
CCCACACCGT CCATGCTGATAA ATAGCTGGCTGGC CAGTGGCACGG TGGGCTGGACATC AATGCTAAAGT
                    -51
```

```
                   1              5                  10                 15
                  MET ALA ILE PRO ASP GLU PHE ASP ILE ILE VAL VAL GLY GLY GLY SER THR
ACAAAAACAAA ATG GCC ATT CCT GAC GAA TTC GAT ATC ATT GTT GTT GGT GGA GCT TCC ACC
-11
        20                 25                 30                 35
GLY CYS CYS ILE ALA GLY ARG LEU ALA ASN LEU ASP ASP GLN ASN LEU THR VAL ALA LEU
GGC TGC TGC ATT GCG GCC AGA CTC GCA AAC CTC GAC GAC CAA AAC CTC ACA GTT GCC CTG
        40                 45                 50                 55
ILE GLU GLY GLY GLU ASN ASN ILE ASN ASN PRO TRP VAL TYR LEU PRO GLY VAL TYR PRO
ATC GAG GGT GGT GAC AAC AAC ATC AAC AAC CCT TGG GTC TAC CTT CCC GGA GTG TAT CCT
        60                 65                 70                 75
ARG ASN MET ARG LEU ASP SER LYS THR ALA THR PHE TYR SER SER ARG PRO SER LYS ALA
AGA AAC ATG AGA CTC GAC TCC AAG ACG GCC ACC TTC TAC TCG TCC AGA CCA TCG AAG GCT
        80                 85                 90                 95
LEU ASN GLY ARG ARG ALA ILE VAL PRO CYS ALA ASN ILE LEU GLY GLY GLY SER SER ILE
CTG AAC GGC AGA AGA GCG ATC GTT CCT TGC GCC AAC ATC CTT GGA GGC GGC TCG TCC ATC
        100                105                110                115
ASN PHE LEU MET TYR THR ARG ALA SER ALA SER ASP TYR ASP ASP TRP GLU SER GLU GLY
AAC TTT CTG ATG TAC ACC AGA GCC TCT GCT TCC GAC TAC GAC GAC TGG GAG TCC GAG GGA
        120                125                130                135
TRP SER THR ASP GLU LEU LEU PRO LEU ILE LYS LYS ILE GLU THR TYR GLN ARG PRO CYS
TGG AGC ACC GAC GAG TTG CTA CCT CTC ATC AAA AAA ATC GAA ACT TAC CAG CCT CCT TGC
        140                145                150                155
ASN ASN ARG ASP LEU HIS GLY PHE ASP GLY PRO ILE LYS VAL SER PHE GLY ASN TYR THR
AAC AAC AGA GAT CTG CAC GGC TTT GAC GGC CCA ATC AAG GTT TCC TTT GGA AAC TAC ACG
        160                165                170                175
TYR PRO THR CYS GLN ASP PHE LEU ARG ALA ALA GLU SER GLN GLY ILE PRO VAL VAL ASP
TAT CCT ACC TGC CAG GAC TTC CTC AGA GCA GCA GAG TCG CAG GGA ATT CCT GTT GTG GAC
        180                185                190                195
ASP LEU GLU ASP PHE LYS THR SER HIS GLY ALA GLU HIS TRP LEU LYS TRP ILE ASN ARG
GAC CTG GAG GAC TTC AAG ACA TCG CAT GGT GCA GAG CAC TGG CTC AAG TGG ATT AAC AGA
        200                205                210                215
ASP LEU GLY ARG ARG SER ASP SER ALA HIS ALA TYR VAL HIS PRO THR MET ARG ASN LYS
GAC CTG GGC AGA AGA TCG GAT TCT GCG CAC GCC TAC GTC CAC CCA ACT ATG AGA AAC AAG
        220                225                230                235
GLN SER LEU PHE LEU ILE THR SER THR LYS CYS ASP LYS VAL ILE ILE GLU ASP GLY LYS
CAG AGC CTG TTC CTC ATC ACC TCC ACC AAG TGT GAC AAG GTG ATC ATC GAG GAC GGC AAG
        240                245                250                255
ALA VAL ALA VAL ARG THR VAL PRO MET LYS PRO LEU ASN PRO LYS LYS PRO VAL SER ARG
GCT GTG GCC GTG AGA ACA GTG CCA ATG AAG CCT CTC AAC CCT AAG AAG CCT GTG TCC AGA
        260                265                270                275
THR PHE ARG ALA ARG LYS GLN ILE VAL ILE SER CYS GLY THR ILE SER SER PRO LEU VAL
ACC TTC AGA GCC AGA AAG CAG ATT GTG ATC TCC TGC GCA ACC ATC TCG TCT CCT CTG GTG
        280                285                290                295
LEU GLN ARG SER GLY ILE GLY ALA ALA HIS HIS LEU ARG SER VAL GLY VAL LYS PRO ILE
CTC CAG AGA TCT GGT ATT GGT GCA GCT CAC CAC TTG AGA TCC GTG GGC GTC AAG CCA ATC
```

33

```
              300                  305                  310                  315
VAL ASP LEU PRO GLY VAL GLY GLN ASN PHE GLN ASP HIS TYR CYS PHE PHE THR PRO TYR     FIG. 4B
GTC GAC CTG CCA GGT GTG GGT GAG AAT TTC CAG GAC CAC TAC TGT TTC TTC ACT CCA TAC
              320                  325                  330                  335
TYR VAL LYS PRO ASP VAL PRO THR PHE ASP ASP PHE VAL ARG GLY ASP PRO VAL ALA GLN
TAC GTC AAG CCT GAC GTT CCT ACG TTC GAC GAC TTT GTC AGG GGC GAC CCA GTT GCC CAG
              340                  345                  350                  355
LYS ALA ALA PHE ASP GLN TRP TYR SER ASN LYS ASP GLY PRO LEU THR THR ASN GLY ILE
AAG GCC GCT TTC GAC CAG TGG TAC TCC AAC AAG GAC GGT CCA TTG ACC ACC AAC GGT ATT
              360                  365                  370                  375
GLU ALA GLY VAL LYS ILE ARG PRO THR GLU GLU GLU LEU ALA THR ALA ASP GLU ASP PHE
GAA GCC GGA GTC AAC ATC AGA CCT ACC GAA GAG GAG CTG GCT ACC GCG GAC GAG GAC TTC
              380                  385                  390                  395
ARG ARG GLY TYR ALA GLU TYR PHE GLU ASN LYS PRO ASP LYS PRO LEU MET HIS TYR SER
AGA CGC GGC TAC GCA GAG TAC TTC GAG AAC AAG CCA GAC AAG CCT CTG ATG CAC TAC TCT
              400                  405                  410                  415
VAL ILE SER GLY PHE PHE GLY ASP HIS THR LYS ILE PRO ASN GLY LYS PHE MET THR MET
GTC ATC TCC GGC TTC TTT GGA GAC CAC ACC AAG ATT CCT AAC GGC AAG TTC ATG ACC ATG
              420                  425                  430                  435
PHE HIS PHE LEU GLU TYR PRO PHE SER ARG GLY PHE VAL ARG ILE THR SER ALA ASN PRO
TTC CAC TTC CTG GAG TAT CCA TTC TCC AGA GGA TTT GTT AGA ATC ACC TCG GCA AAC CCA
              440                  445                  450                  455
TYR ASP ALA PRO ASP PHE ASP PRO GLY PHE LEU ASN ASP GLU ARG ASP LEU TRP PRO MET
TAC GAC GCT CCT GAC TTC GAT CCC GGC TTC CTC AAT GAC GAA AGA GAC CTG TGG CCT ATG
              460                  465                  470                  475
VAL TRP ALA TYR LYS LYS SER ARG GLU THR ALA ARG ARG MET GLU SER PHE ALA GLY GLU
GTC TGG GCA TAC AAG AAG TCC AGA GAG ACG GCC AGA AGA ATG GAG AGC TTT GCA GGA GAG
              480                  485                  490                  495
VAL THR SER HIS HIS PRO LEU PHE LYS VAL ASP SER PRO ALA ARG ALA ARG ASP LEU ASP
GTC ACC TCG CAC CAC CCA TTG TTC AAG GTT GAC TCG CCA GCC AGA GCC AGA GAC CTG GAC
              500                  505                  510                  515
LEU GLU THR CYS SER ALA TYR ALA GLY PRO LYS HIS LEU THR ALA ASN LEU TYR HIS GLY
CTC GAG ACA TGC AGT GCA TAT GCC GGT CCT AAG CAC CTC ACT GCC AAC CTC TAC CAC GGC
              520                  525                  530                  535
SER TRP THR VAL PRO ILE ASP LYS PRO THR PRO LYS ASN ASP PHE HIS VAL THR SER ASN
TCG TGG ACC GTT CCT ATC GAC AAG CCA ACG CCT AAG AAC GAT TTC CAC GTG ACC TCC AAC
              540                  545                  550                  555
GLN VAL GLN LEU HIS SER ASP ILE GLU TYR THR GLU GLU ASP ASP GLU ALA ILE VAL ASN
CAA GTC CAA CTG CAC TCC GAC ATC GAG TAC ACC GAG GAG GAC GAC GAG GCC ATC GTC AAC
              560                  565                  570                  575
TYR ILE LYS GLU HIS THR GLU THR THR TRP HIS CYS LEU GLY THR CYS SER MET ALA PRO
TAC ATT AAG GAA CAC ACC GAG ACC ACT TGG CAC TGT CTG GGT ACC TGC TCG ATG GCC CCA
              580                  585                  590                  595
ARG GLU GLY SER LYS ILE ALA PRO LYS GLY GLY VAL LEU ASP ALA ARG LEU ASN VAL TYR
AGA GAG GGT AGT AAG ATT GCT CCT AAG GCA GGT GTC TTG GAC GCC AGA CTC AAC GTT TAC
              600                  605                  610                  615
GLY VAL GLN ASN LEU LYS VAL ALA ASP LEU SER VAL CYS PRO ASP ASN LEU GLY CYS-ASN
GGA GTC CAG AAC CTC AAG GTT GCC GAC CTT TCT GTT TGT CCC GAC AAC CTT GGA TGC AAC
              620                  625                  630                  635
THR TYR SER THR ALA LEU THR ILE GLY GLU LYS ALA ALA THR LEU VAL ALA GLU ASP LEU
ACC TAC TCT ACT GCA TTG ACC ATC GGT GAG AAG GCT GCC ACT CTT GTT GCT GAA GAT CTT
              640                  645                  650                  655
GLY TYR SER GLY SER ASP LEU ASP MET THR ILE PRO ASN PHE ARG LEU GLY THR TYR GLU
GGC TAC TCA GGC TCC GAC CTC GAC ATG ACC ATT CCA AAC TTC AGA CTC GGA ACT TAC GAG
              660
GLU THR GLY LEU ALA ARG PHE ***
GAG ACC GGA CTT GCC AGA TTC TAA GGAG ACCTGGAAGG ACATACCCCT TTTGAGAAGC
                                                     2000
        GTGTTTGAAA ATAGTTCTTT TTCTGGTTTA TATCGTTTAT GAAGTGATGA GATGAAAAGC
                                        2050
        TGAAATAGCG AGTATAGGAA AATTTAATGA AAATTAAATT AAATATTTTC TTAGGCTATT
        AGTCACCTTC AAAATGCCGG CCGCTTCTAA GAACGTTGTC ATGATCGACA ACTACGACTC
   2150                                                              2200
        GTTTACCTGG AACGTGTACC AGTACCTGTG TCAGGAGGGA GCCAATGTCG AGGTTTTCAG
                                                     2250
        GAACGATCAG ATCACCATTC GGGACGATTGA GCAGCTCAAG CCCGACGTTG TGGTGATATC
                                        2300
        CCCTGGTCCT GGGCATCCAA GAAChAGACTC GGGAATATCT CGCCACGTGA TCAGCCATTT
                                        2350
        TAAAGGCAAG ATTCCTGTCT TTGGTGTCTG TATGGGCCAG CAGTGTATCT TCGAGGAGTT
                          2400
        TGGCGGAGAC GTCGAGTATC CGGCCCAGAT TGTCCATGGA AAAACGTCCA CTGTTAAGCA
   2450                                                 2500
        CGACAACAAG GGAATGTTCA AAAACGTTCC GCAAGATGTT GCTGTCACCA GATACCACTC
                                                     2550
        GCTGCCCGCA ACCCTCAAGT CGCTTCCGCA CTGTCTAGAG ATCACTGCTC GCACAGACAA
                                        2600
        CGGCATCATT ATGGGTGTGA GACACAAGAA GTACACCATC GAGGCCGTCC AGTTTCATCC
                          2650
        AGAGAGGCATT CTGACCGAGG AGGGCCATCT GATGATCCAG AATATCCTCA ACGTTTCCGG
                     2700
        TGGTTACTGG GAGGAAAATG CCAACGGCGC GGCTCAGACA AAGGAAAGCA TATTGGAGAA
   2750                                                 2800
        AATATACGCG GAGAGACGAA AAGACTACGA GTTTGAGATG AACAGACCGG GGCGCACATT
                                        2850
        TGCTGATCTA GAACTGTACT TGTCCATGGG ACTGCACCGC GGCTAATCAA TTTTTACGAC
   2900
        AGATTGGAGC AGAACATCAG CGCCGGCAAG GTTGCAATTC TCAGCGAAAT CAACAGAGCG
                                        2950
        TGGCCTTCTA AAGGCGTGAT CGACGCAGAC GCTAACGCTG CCAAACAGGC CCTCAACTAC
                     3000
        GCCAAGGCTG GACTTCCCAC AATTTCTGTT TTCACCGAGC GAACCTGGTT TAAAGGCAAAT
   3050                                                 3100
        ATCCAGGACC TGGAGGTCGC CAGAAAACCC ATTGACTCTG TGGCCAATAC ACCCTGTATT
                                        3150
        TTGCGGAAGG AGTTTATCTT CAACAAGTAC CAAATTCTAG AGGCCCCACT GGCCGGCAGCA
                                3200
        GACACGGTTC TGCTGATTGT CAAGATGCTG AGCTC
                          3250
```

Fig. 12A

λMOX4 (fig.6) Transposon Tn5 M13 mp9

↓ Sal I ↓ Sal I ↓ Sal I alkaline phosphatase

isolate 2.4 kb fragment isolate 1.1 kb fragment alcohol precipitation

↓ Xma III ↓ Xma III

isolate 380 bp fragment

T4 ligase

Xma III Sma I

Sal I Sal I

Hin dIII pr. MOX NEO

pUR 3101

λMOX 4 (fig. 6) 5'TGGC —NEO3— —NEO7— CTCCGGCCGCTTG 3' M13 mp9

3'ACGTGCCA————————GAGGCCGGCGAACTTAA 5'

↓ Sal I Hgi AI NEO6 NEO8 Xma III EcoRI ↓ Sal I EcoRI

isolate 2.4 kb fragment phosphorylate NEO 6 and 7 with T4 polynucleotide kinase and rATP isolate vector

↓ Hgi AI NEO 3, 6, 7 and 8 ↓ T4 ligase

isolate 1.5 kb fragment isolate 86 bp fragment

↓ T4 polynucleotide kinase and rATP

T4 ligase

Xma III Hgi AI

Sal I Xma III

Hin dIII pr. MOX EcoRI

NEO

pUR 3102

Fig. 12B

Promoter MOX-Neomycinphosphotransferase adaptor fragments

NEO3  5'CGGTGGTGACATCAATCTAAAGTACAAA 3'

NEO6  5'TCATTTTGTTTTTGTACTTTAGATTGATGTCACCACCGTGCA 3'

NEO7 5'AACAAAATGATTGAACAAGATGGATTGCACGCAGGTTCTCCGGCCGCTTG 3'

NEO8 5'AATTCAAGCGGCCGGAGAACCTGCGTGCAATCCATCTTGTTCAA 3'

Fig. 12C

Fig. 13

```
<---------------- PROMOTER MOX/AAO ADAPTOR---------------------->>
   -34                                   1
     CGGTGG TGACATCAAT CTAAAGTACA AAAACAAAAT GAGAGTTGTC GTTATTGGTG
ACGTGCCACC ACTGTAGTTA GATTTCATGT TTTTGTTTTA CTCTCAACAG CAATAACCAC
HgiaI                                   Met


<---------------->

                                        62
CCCGTGTCAT CGGTCTGTCG ACCGCCCTGT GTATCCACGA GAGATACCAC TCCGTTCTGC
GGCCACAGTA GCCAGACAGC TGGCGGGACA CATAGGTGCT CTCTATGGTG AGGCAAGACG
            . SalI


AGCCTCTGGA CGTTAAGGTC TACGCCGACA GATTCACCCC TTTCACCACC ACCGACGTTG
TCGGAGACCT GCAATTCCAG ATGCGGCTGT CTAAGTGGGG AAAGTGGTGG TGGCTGCAAC


CCGCCGGTCT GTGGCAGCCT TACACCTCCG AGCCTTCCAA CCCTCAGGAG GCCAACTGGA
GGCGGCCAGA CACCGTCGGA ATGTGGAGGC TCGGAAGGTT GGGAGTCCTC CGGTTGACCT


ACCAGCAGAC CTTCAACTAC CTCCTCTCCC ACATCGGTTC GCCTAACGCC GCCAACATGG
TGGTCGTCTG GAAGTTGATG GAGGAGAGGG TGTAGCCAAG CGGATTGCGG CGGTTGTACC


GTCTGACCCC TGTCTCGGGT TACAACCTGT TCAGAGAGGC CGTTCCTGAC CCTTACTGGA
CAGACTGGGG ACAGAGCCCA ATGTTGGACA AGTCTCTCCG GCAAGGACTG GGAATGACCT


AGGACATGGT CCTCGGTTTC AGAAAGCTTA CCCCTAGAGA GCTGGACATG TTCCCTGACT
TCCTGTACCA GGAGCCAAAG TCTTTCGAAT GGGGATCTCT CGACCTGTAC AAGGGACTGA
                      HindIII


ACAGATACGG TTGGTTCAAC ACCTCCCTGA TCCTGGAGCG TAGAAAGTAC CTGCAGTGGC
TGTCTATGCC AACCAAGTTG TGGAGGGACT AGGACCTCCC ATCTTTCATG GACGTCACCG


TGACCGAGAG ACTGACCGAG AGAGGTGTTA AGTTCTTCCT GAGAAAGCTC GAGTCCTTCG
ACTGGCTCTC TGACTGGCTC TCTCCACAAT TCAAGAAGGA CTCTTTCCAG CTCAGGAAGC


AGGAGGTTGC CAGAGGTGGT GCCGACGTCA TCATCATGTG TACCGGTGTC TGGGCCGGTG
TCCTCCAACG GTCTCCACCA CGGCTGCAGT AGTAGTACAC ATGGCCACAG ACCCGGCCAC


TCCTGCAGCC TGACCCTCTC CTGCAGCCCC GGAGAGGTCA GATCATTAAG GTTGACGCCC
AGGACGTCGG ACTGGGAGAC GACGTCGGGC CCTCTCCAGT CTAGTAATTC CAACTGCGGG
                      XmaI


CATGGCTGAA GAACTTCATC ATTACCCACG ACCTGGAGAG AGGTATCTAC AACTCCCCTT
GTACCGACTT CTTGAAGTAG TAATGGGTGC TGGACCTCTC TCCATAGATG TTGAGGGGAA


ACATTATCCC TGGTCTGCAG GCCGTCACCC TGGGTGGTAC CTTCCAGGTC GGTAACTGGA
TGTAATAGGG ACCAGACGTC CGGCAGTGGG ACCCACCATG GAAGGTCCAG CCATTGACCT
                                      KpnI


ACGAGATCAA CAACATCCAG GACCACAACA CCATCTGGGA GGGTTGTTGT AGACTGGAGC
TGCTCTAGTT GTTGTAGGTC CTGGTGTTGT GGTAGACCCT CCCAACAACA TCTGACCTCG


CTACCCTGAA GGACGCCAAG ATCGTTGGTG AGTACACCGG TTTCAGACCT GTTAGACCTC
GATGGGACTT CCTGCGGTTC TAGCAACCAC TCATGTGGCC AAAGTCTGGA CAATCTGGAG


AGGTCAGACT GGAGAGAGAG CAGCTGAGAT TCGGTTCCTC CAACACCGAG GTCATTCACA
TCCAGTCTGA CCTCTCTCTC GTCGACTCTA AGCCAAGGAG GTTGTGGCTC CAGTAAGTGT


ACTACGGTCA CGGTGGTTAC GGTCTGACCA TCCACTTGGG TTGTGCCCTG GAGGTTGCCA
TGATGCCAGT GCCACCAATG CCAGACTGGT AGGTGAACCC AACACGGGAC CTCCAACGGT


AGCTGTTCGG TAAGGTCCTG GAGGAGAGAA ACCTGCTGAC CATGCCTCCA TCCCACCTGT
TCGACAAGCC ATTCCAGGAC CTCCTCTCTT TGGACGACTG GTACGGAGGT AGGGTGGACA
                                                                T


GAG
CTCAGCT
**SalI
```

Fig. 14A

Fig. 143

## Fig. 14C

<------PROMOTER MOX-HGRF ADAPTOR------->>
                    -34
                CGGTG GTGACATCAA TCTAAAGTA CAAAAACAAA
              ACGTGCCAC CACTGTAGTT AGATTTCAT GTTTTTGTTT
              HgiAI

<<----------------------------------------------------------------->>
1
ATGTACGCCG ACGCCATCTT CACCAACTCC TACAGAAAGG TTCTGGGTCA GCTCTCGGCC
TACATGCGGC TGCGGTAGAA GTGGTTGAGG ATGTCTTTCC AAGACCCAGT CGAGAGCCGG
Met

--->
61
AGAAAGCTTC TGCAGGACAT CATGTCGAGA CAGCAGGGTG AGTCCAACCA GGAGAGAGGT
TCTTTCGAAG ACGTCCTGTA GTACAGCTCT GTCGTCCCAC TCAGGTTGGT CCTCTCTCCA
   HindIII PstI

121
GCCAGAGCCA GACTGTGAG
CGGTCTCGGT CTGACACTCA GCT                    Fig. 15
              *** SalI

Fig. 16A

Fig. 16B

Fig. 16C

Fig. 16D

Fig. 16E

EP 0 423 890 A2

Fig. 16F

pURS 528-03    pUR 3209

| Hpa I
| Sal I
| klenow
| polymerase

| partial Kpn I
| klenow
| polymerase

isolate Leu 2
containing fragment

T4 ligase

pUR 3211

transformation

EP 0 423 890 A2

```
1
CATGTACGCCG ACGCCATCTT CACCAACTCC TACAGAAAGG TTCTGGGTCA GCTCTCGGCC
CATGGTACATGCGGC TGCGGTAGAA GTGGTTGAGG ATGTCTTTCC AAGACCCAGT CGAGAGCCGG
KpnI Met


61
AGAAAGCTTC TGCAGGACAT CTGTTCGAGA CAGCAGGGTG AGTCCAACCA GGAGAGAGGT
TCTTTCGAAG ACGTCCTGTA GACAAGCTCT GTCGTCCCAC TCAGGTTGGT CCTCTCTCCA
  HindIII PstI           cys


121
GCCAGAGCCA GACTGTGAGGTAC
CGGTCTCGGT CTGACACTC
              *** KpnI
```

Fig. 17

## Fig. 18A

```
                                                        G GATCCACCTG
                                                          -2125
CTTGGCCAAT GATTCAGCTG CTGGACCGAA AACGCCTCTT TTGGCCAAAA AAAGCCCACC
 -2104
GTTGATAACT GCGGAGGCCA TATTTCAAAG AACAGCGAAT AACAAAAAAA GGTGAATGAA
 -2054                                                     -2004
ATGCGCGAAA CGATACCACT TATTAGCATA AACAAAAAAA AAAAAAATCT ATTAGCTGTT
                                             -1954
ATTATAATTA GTTCAATAAT TTCATAAGCA TCATGGTTGG GCGGCCTATT GTCATCAGTG
                                    -1904
GTCCCTCTGG AACAGGTAAA TCCACTTTGC TGAAGAAGCT GTTTGCTGAG TTCCCAGACA
                          -1854
AGTTTGGATT TTCCGTGTCC AACACCACGA GAAAACCTAG ACCTGGTGAA AAAGACGGTG
           -1804
TCGATTACCA CTTCACCACG GTAGAGGACT TCAAGAAGAT GATTGAAGAA AACAAATTCA
-1754                                                      -1704
TTGAATGGGC CCAGTTCTCC GGCAACTACT ACGGCACCTC TGTGAAAGCT GTGCAAGACG
                                           -1654
TGGCCGAACT GATGAAGAGA ACGTGTATTT TGGACATTGA TATGCAGGGT GTCAAGAGCG
                                  -1604
TCAAGAAGAC CAACCTGGCA GCCCGATTCC TCTTTATTTC TCCTCCGTCC ATCGAAGAGC
                -1554
TCAAGAAGAG GCTCGAGAGC CGTGGAACAG AGACCCCTGA ATCTCTTGCC AAGCGGCTTG
           -1504
CTGCTGCATC TGCGGAGATG GAGTACGCCA GGGCAGTGGA CACGACAAGG TCATTGTCAA
-1454                                                      -1404
CGATGACCTT GAGAAGGCGT ACTCTGAGCT GAAGGAGTTC ATTTTCGCCG AGCCCATCTA
                                           -1354
AGCATTCATA AATTTTTAAT ATCTAGAGCT CTCATACGGG ACAGTATCTC CTCCAACCTT
                                  -1304
GCGTCAAGCT TGTCCTCTTC ATGCTCCTCA ACAGTCATGG CATCCAGCTG CTGCTGCTTT
                -1254
TGCTCCAGCC TGGCATATAT GTCGCCATAC AGCTTGAGTT GGATTTTGAT GAAACTCTCA
           -1204
AAGGTAGGGT CCACCAGTGA CAGTCGCAGC GCAATGAACT GCTCGATTTC GTTCTTGAGC
-1154                                                      -1104
CGTGTGTTGA TGTCCGTGTA GATATTTTCT GCCTCGTCGT ACTCAACTTT GAACTTCTGC
                                           -1054
AGCTTGTCCA GGCTCTTCTG TAACTGGTCT GTTTTCTCGG TGTGATGCTG CTCGGTCACC
                -1004
TGTCGCTCAA TCGCTTCGTA CTCGCTCTGC AGCTTCGAAA GCTTGAATCG TGAAACGTCG
           -954
TAATCCACCT TTTTGCGTGC GCGCTTCTTG ATCAGCTTGT TGATCTCGTC GTTGTACTTC
           -904
TTCAGCTCGT TAATCGGCTC CACGACCGTG ATGCTCATTG GCTCCAGAAT TTCTGGCAGA
-854                                                       -804
ATATTGTCTT TGATGTCTTC CACCATCTGC AGATAATTCA GAGAAATACC ATCTCTGGGG
                                           -754
TTCACCTTGT GCTCTTCTGG CCGTTCCGCA GCTTCCGACC GCTTATCAGC CTTGAGCTCA
                                  -704
AAGCTATAGT CTCCGTAAAA CGAGTCCAGT GTTCTAGCCA TATTTATCTG AGTCTCGAGC
                          -654
AGATTCTCCG AAAATTGCCCA CAAAACGGCC TAGTTCCTGG TCCAGCTCGT TGGTGTAAGT
           -604
CTCGAGTTTG CGGAAATTGG CCTCCTGGAC GTCAAACTCA GGATCAACAG AGGGCTCACC
-554                                                       -504
TTTGTTTGTG CGTAGTATCA CATGTGCTCC GGCACGATTG ACAGCTTTTT TAAACCCAAC
                                           -454
CCATGACATG TCGAGGAAAG GGTCGTTTCG GGGAGTTAAA TATTTTTGGC TATGTAGCAG
                          -404
ACATGTTTCG ACGCTGGCGT CGCGTCGATC GGAAAATATT ACCCCAGGAA CAAGCACTTG
           -354
CTTGGGTTAG CCACCACCCT GCGCAAGCCT TTTTGCCGGC TCTACACAGG GCCAATGAAA
           -304
TCTGGGCGGA ATCTGAAACC GATGAAACGG ACGACACTGC CAACAAGCTC ACTGCACTAT
-254                                                       -204
```

Fig. 183

```
TTTTTTTTTC TAGTGAAATA GCCTATCCTC GTCTCGCTCC CCTCATACCT GTAAAGGGGT
                                                          -154
GCAATTTAGC CTCGTTCCAG CCATTCACGG GCCACTCAAC AACACGTCGG CTACCATGGG
                                                          -104
GTGCTTGGGC ACCAAAAGGC CTATAAATAG GCCCCCATCC GTCTGCTACA CAGTCATCTC
                                                          -54
```

```
          1                      5                      10                     15
          MET SER MET ARG ILE PRO LYS ALA ALA SER VAL ASN ASP GLU GLN HIS
TGTCTTTTCTTCCC ATG AGT ATG AGA ATC CCT AAA GCA GCG TCG GTC AAC GAC GAA CAA CAC
-14
          20                      25                     30                     35
GLN ARG ILE ILE LYS TYR GLY ARG ALA LEU VAL LEU ASP ILE VAL GLU GLN TYR GLY GLY
CAG AGA ATC ATC AAG TAC GGT CGT GCT CTT GTC CTG GAC ATT GTC GAG CAG TAC GGA GGA
              40                      45                     50                     55
GLY HIS PRO GLY SER ALA MET GLY ALA MET ALA ILE GLY ILE ALA LEU TRP LYS TYR THR
GGC CAC CCG GGC TCG GCC ATG GGC GCC ATG GCT ATC GGA ATT GCT CTG TGG AAA TAC ACC
LEU LYS TYR ALA PRO ASN ASP PRO ASN TYR PHE ASN ARG ASP ARG PHE VAL LEU SER ASN
CTG AAA TAT GCT CCC AAC GAC CCT AAC TAC TTC AAC AGA GAC AGG TTT GTC CTG TCG AAC
              80                      85                     90                     95
GLY HIS VAL CYS LEU PHE GLN TYR ILE PHE GLN HIS LEU TYR GLY LEU LYS SER MET THR
GGT CAC GTG TGT CTG TTC CAG TAT ATC TTC CAG CAC CTG TAC GGT CTC AAG TCG ATG ACC
          100                     105                    110                    115
MET ALA GLN LEU LYS SER TYR HIS SER ASN ASP PHE HIS SER LEU CYS PRO GLY HIS PRO
ATG GCG CAG CTG AAG TCC TAC CAC TCG AAT GAC TTC CAC TCG CTG TGT CCC GGT CAC CCA
GLU ILE GLU HIS ASP ALA VAL GLU VAL THR THR GLY PRO LEU GLY GLN GLY ILE SER ASN
GAA ATC GAG CAC GAC GCC GTC GAG GTC ACA ACG GGC CCG CTC GGC CAG GGT ATC TCG AAC
          140                     145                    150                    155
SER VAL GLY LEU ALA ILE ALA THR LYS ASN LEU ALA ALA THR TYR ASN LYS PRO GLY PHE
TCT GTT GGT CTG GCC ATA GCC ACC AAA AAC CTG GCT GCC ACG TAC AAC AAG CCG GGC TTT
          160                     165                    170                    175
ASP ILE ILE THR ASN LYS VAL TYR CYS MET VAL GLY ASP ALA CYS LEU GLN GLU GLY PRO
GAT ATC ATC ACC AAC AAG GTG TAC TGC ATG GTT GGC GAT GCG TGC TTG CAG GAG GGC CCT
          180                     185                    190                    195
ALA LEU GLU SER ILE SER LEU ALA GLY HIS MET GLY LEU ASP ASN LEU ILE VAL LEU TYR
GCT CTC GAG TCG ATC TCG CTG GCC GGC CAC ATG GGG CTG GAC AAT CTG ATT GTG CTC TAC
          200                     205                    210                    215
ASP ASN ASN GLN VAL CYS CYS ASP GLY SER VAL ASP ILE ALA ASN THR GLU ASP ILE SER
GAC AAC AAC CAG GTC TGC TGT GAC GGC AGT GTT GAC ATT GCC AAC ACG GAG GAC ATC AGT
          220                     225                    230                    235
ALA LYS PHE LYS ALA CYS ASN TRP ASN VAL ILE GLU VAL GLU ASN ALA SER GLU ASP VAL
GCC AAG TTC AAG GCC TGC AAC TGG AAC GTG ATC GAG GTC GAG AAC GCT TCC GAG GAC GTG
          240                     245                    250                    255
ALA THR ILE VAL LYS ALA LEU GLU TYR ALA GLN ALA GLU LYS HIS ARG PRO THR LEU ILE
GCT ACC ATT GTC AAG GCC TTG GAG TAC GCG CAG GCC GAG AAG CAC AGA CCA ACA CTT ATC
          260                     265                    270                    275
ASN CYS ARG THR VAL ILE GLY SER GLY ALA ALA PHE GLU ASN HIS CYS ALA ALA HIS GLY
AAC TGC AGA ACT GTC ATT GGA TCG GGT GCT GCG TTC GAG AAC CAC TGT GCT GCG CAC GGT
          280                     285                    290                    295
ASN ALA LEU GLY GLU ASP GLY VAL ARG GLU LEU LYS ILE LYS TYR GLY MET ASN PRO ALA
AAC GCT CTG GGC GAG GAC GGT CTC CGC GAG CTC AAA ATC AAG TAC GGC ATG AAC CCG GCC
          300                     305                    310                    315
GLN LYS PHE TYR ILE PRO GLN ASP VAL TYR ASP PHE PHE LYS GLU LYS PRO ALA GLU GLY
CAG AAG TTC TAC ATT CCG CAG GAC GTG TAC GAC TTC TTC AAG GAG AAG CCG GCC GAG GGC
          320                     325                    330                    335
ASP LYS LEU VAL ALA GLU TRP LYS SER LEU VAL ALA LYS TYR VAL LYS ALA TYR PRO GLU
GAC AAG CTG GTG GCC GAA TGG AAG AGT CTC GTG GCC AAG TAC GTC AAG GCG TAC CCT GAG
          340                     345                    350                    355
GLU GLY GLN GLU PHE LEU ALA ARG MET ARG GLY GLU LEU PRO LYS ASN TRP LYS SER PHE
GAG GGC CAG GAG TTT TTG GCG CGG ATG AGA GGC GAG CTG CCA AAG AAC TGG AAG TCG TTC
          360                     365                    370                    375
```

EP 0 423 890 A2

Fig. 18C

```
LEU PRO GLN GLN GLU PHE THR GLY ASP ALA PRO THR ARG ALA ALA ALA ARG GLU LEU VAL
CTG CCG CAG CAG GAA TTC ACC GGC GAC GCT CCT ACA AGG GCC GCT GCC AGA GAG CTT GTG
            380                    385                    390                    395
ARG ALA LEU GLY GLN ASN CYS LYS SER VAL ILE ALA GLY CYS ALA ASP LEU SER VAL SER
AGA GCC CTG GGG CAG AAC TGC AAG TCG GTG ATT GCC GGT TGC GCA GAC CTG TCT GTG TCT
            400                    405                    410                    415
VAL ASN LEU GLN TRP PRO GLY VAL LYS TYR PHE MET ASP PRO SER LEU SER THR GLN CYS
GTC AAT TTG CAG TGG CCA GGG GTG AAA TAT TTC ATG GAC CCC TCG CTG TCC ACG CAG TGT
            420                    425                    430                    435
GLY LEU SER GLY ASP TYR SER GLY ARG TYR ILE GLU TYR GLY ILE ARG GLU HIS ALA MET
GGC CTG AGC GGC GAC TAC TCC GGC AGA TAC ATT GAG TAC GGA ATC AGA GAA CAC GCC ATG
            440                    445                    450                    455
CYS ALA ILE ALA ASN GLY LEU ALA ALA TYR ASN LYS GLY THR PHE LEU PRO ILE THR SER
TGT GCT ATC GCC AAT GGC CTT GCC GCC TAC AAC AAG GGC ACG TTC CTG CCG ATC ACG TCG
            460                    465                    470                    475
THR PHE PHE MET PHE TYR LEU TYR ALA ALA PRO ALA ILE ARG MET ALA GLY LEU GLN GLU
ACT TTC TTC ATG TTC TAC CTG TAC GCT GCC CCA GCC ATC AGA ATG GCC GGC CTG CAG GAG
            480                    485                    490                    495
LEU LYS ALA ILE HIS ILE GLY THR HIS ASP SER ILE ASN GLU GLY GLU ASN GLY PRO THR
CTC AAG GCG ATC CAC ATC GGC ACC CAC GAC TCG ATC AAT GAG GGT GAG AAC GGC CCT ACG
            500                    505                    510                    515
HIS GLN PRO VAL GLU SER PRO ALA LEU PHE ARG ALA TYR ALA ASN ILE TYR TYR MET ARG
CAC CAG CCG GTC GAG TCG CCA GCA TTG TTC CGG GCC TAT GCA AAC ATT TAC TAC ATG AGA
            520                    525                    530                    535
PRO VAL ASP SER ALA GLU VAL PHE GLY LEU PHE GLN LYS ALA VAL GLU LEU PRO PHE SER
CCG GTC GAC TCT GCA GAA GTG TTT GGC CTG TTC CAA AAA GCC GTC GAG CTG CCA TTC AGC
            540                    545                    550                    555
SER ILE LEU SER LEU SER ARG ASN GLU VAL LEU GLN TYR LEU ALA SER ARG ALA GLN ARG
TCG ATT CTG TCG CTC TCG AGA AAC GAG GTG CTG CAA TAC CTG GCA AGT CGA GCG CAG AGA
            560                    565                    570                    575
ARG ARG ASN ALA ALA GLY TYR ILE LEU GLU ASP ALA GLU ASN ALA GLU VAL GLN ILE ILE
AGG CGC AAC GCG GCC GGC TAT ATT CTG GAG GAT GCG GAG AAC GCC GAG GTG CAG ATT ATT
            580                    585                    590                    595
GLY VAL GLY ALA GLU MET GLU PHE ALA ASP LYS ALA ALA LYS ILE LEU GLY ARG LYS PHE
GGA GTT GGT GCA GAG ATG GAG TTT GCA GAC AAG GCC GCC AAG ATC TTG GGC AGA AAG TTC
            600                    605                    610                    615
ARG THR ARG VAL LEU SER ILE PRO CYS THR ARG LEU PHE ASP GLU GLN SER ILE GLY TYR
AGG ACC AGA GTT CTC TCC ATC CCA TGC ACG CGG CTG TTT GAC GAG CAG TCG ATC GGC TAT
            620                    625                    630                    635
ARG ARG SER VAL LEU ARG LYS ASP GLY ARG GLN VAL PRO THR VAL VAL VAL ASP GLY HIS
AGA CGC TCG GTT TTG AGA AAG GAC GGC AGA CAG GTG CCA ACG GTG GTG GTG GAC GGC CAC
            640                    645                    650                    655
VAL ALA PHE GLY TRP GLU ARG TYR ALA THR ALA SER TYR CYS MET ASN THR TYR GLY LYS
GTT GCG TTC GGC TGG GAG AGA TAC GCT ACG GCG TCC TAC TGT ATG AAC ACG TAC GGC AAG
           · 660                    665                    670                    675
SER LEU PRO PRO GLU VAL ILE TYR GLU TYR PHE GLY TYR ASN PRO ALA THR ILE ALA LYS
TCT CTG CCT CCA GAA GTG ATC TAC GAG TAC TTT GGA TAC AAC CCG GCA ACG ATT GCC AAG
            680                    685                    690                    695
LYS VAL GLU ALA TYR VAL ARG ALA CYS GLN ARG ASP PRO LEU LEU LEU HIS ARG LEU PRO
AAG GTC GAA GCG TAC GTC CGG GCG TGC CAA AGA GAC CCT TTG CTG CTC CAC CGA CTT CCT
            700
GLY PRO GLU GLY LYS ALA ***
GGA CCT GAA GGA AAA GCC TAA CCACGAT AAAGTAAATA AGCTCTGATT AAGTAAGATG
                                   2110
        AATAAGTTCT TTGTCTGTGA ATGCCACCCC ACAATAACCC CACAAATAAA ACTTTCACAC
        2160                                                       2210
        TTGCGTCAGA AACTGTCGAG CCGCACGGGA CTGACTGTTT GGCGGCCGTGC CTCTGTCCCC
                                                       2260
        ACACGGATAT TTCGCACGGA ACAGAAACCA TTGGACAAGG GGTTGCTGCC GATACCAAAT
                                                       2310
        AGAATGCATC GGATCC
        2350
```

52

Fig. 19

## Fig. 20

### Identical sequences in -1000 region of DAS and MOX genes

DAS -1076

    TAGATATTTTCTGCCTCGTCGTACTCA-54N-GTGTGATG-8N-TCACC-9N-
    \* \*\* \*\*\*\*\* \*\*\*\*\*\*\*\*\*\*\*\*\* \*      \*\*\*\*\*\*\*\*    \*\*\*\*\*


         \* \*\* \*\*\*\*\*\*\*\* \*\*\*\*\*\*\*\*\*\*\*\*\*\*\*\*\*\*\*\*\*\*
    TCGAAATTTTTGCCGTCGTCGTACAGTGTGATGTCACC
  MOX -1052

   DAS                       -937
       ATCGCTTCGTACTCGCTCTGCAGCTTCGA
       \*\*\*\*   \* \*\*\* \* \*   \*\*\*\*\*\*\*\*\*\*\*


       \*\*\*\*    \*\*\*\* \*\*   \*\*\*\*\*\*\*\*\* \*\*\*
       ATCGAATGTAATGAGCTGCAGCTTGCGA
  MOX                     -987